# EUROPEAN PATENT SPECIFICATION

(11) **EP 2 691 111 B1**
(45) Date of publication and mention of the grant of the patent: **20.09.2017**
(21) Application number: 12712273.7
(22) Date of filing: 02.04.2012
(51) Int. Cl.: A61K 39/02, C12Q 1/68, G01N 33/68

(54) **USE OF BLOOD OR TISSUE BACTERIOME FOR PREDICTION, DIAGNOSIS AND PREVENTION OF METABOLIC DISEASES AND THEIR CARDIOVASCULAR COMPLICATIONS**
VERWENDUNG VON BACTERIOME AUS BLUT ODER GEWEBE FÜR VORHERSAGE, DIAGNOSE UND PRÄVENTION VON STOFFWECHSELKRANKHEITEN UND IHRE KARDIOVASKULÄREN KOMPLIKATIONEN
UTILISATION DE BACTERIOME DU SANG OU DE TISSU POUR LA PRÉDICTION, LE DIAGNOSTIC ET LA PREVENTION DES MALADIES METABOLIQUES ET LEURS COMPLICATIONS CARDIOVASCULAIRES

(30) Priority: 31.03.2011 EP 11305366
(43) Date of publication of application: 05.02.2014
(73) Proprietor: Institut National de la Santé et de la Recherche Médicale (INSERM), 75013 Paris (FR); CHU de Toulouse, 31059 Toulouse Cedex 9 (FR)
(72) Inventor: BURCELIN, Rémy, F-31432 Toulouse Cedex 4 (FR); AMAR, Jacques, F-31000 Toulouse (FR); GARIDOU, Lucile, BP 84225, 31432 Toulouse Cedex4 (FR); CHABO, Chantal, 82600 Verdun sur Garonne (FR)
(74) Representative: Lavoix
(86) International application number: PCT/EP2012/055983
(87) International publication number: WO 2012/131099

(56) References cited:
- EP-A1- 2 030 623
- EP-A1- 2 085 466
- EP-A1- 2 216 035
- WO-A2-2010/092164
- US-A1- 2004 197 352
- US-A1- 2010 172 874
- JIA WEI ET AL: "Gut microbiota: a potential new territory for drug targeting.", NATURE REVIEWS. DRUG DISCOVERY FEB 2008 LNKD- PUBMED:18239669, vol. 7, no. 2, February 2008 (2008-02), pages 123-129, XP002657562, ISSN: 1474-1784
- KALLIOMAKI MARKO ET AL: "Early differences in fecal microbiota composition in children may predict overweight", AMERICAN JOURNAL OF CLINICAL NUTRITION, vol. 87, no. 3, March 2008 (2008-03), pages 534-538, XP002657563, ISSN: 0002-9165
- NADJA LARSEN ET AL: "Gut Microbiota in Human Adults with Type 2 Diabetes Differs from Non-Diabetic Adults", PLOS ONE, vol. 5, no. 2, 1 January 2010 (2010-01-01) , pages E9085-E9085, XP055004750, ISSN: 1932-6203, DOI: 10.1371/journal.pone.0009085
- RAMADASS BALAMURUGAN ET AL: "Quantitative differences in intestinal Faecalibacterium prausnitzii in obese Indian children", BRITISH JOURNAL OF NUTRITION, vol. 103, no. 03, 1 February 2010 (2010-02-01), pages 335-338, XP055001765, ISSN: 0007-1145, DOI: 10.1017/S0007114509992182
- KINROSS JAMES M ET AL: "The human gut microbiome: implications for future health care.", CURRENT GASTROENTEROLOGY REPORTS AUG 2008 LNKD- PUBMED:18627653, vol. 10, no. 4, August 2008 (2008-08), pages 396-403, XP002657564, ISSN: 1534-312X
- SUZUKI TOSHIHIKO ET AL: "A novel caspase-1/toll-like receptor 4-independent pathway of cell death induced by cytosolic Shigella in infected macrophages", JOURNAL OF BIOLOGICAL CHEMISTRY, vol. 280, no. 14, April 2005 (2005-04), pages 14042-14050, ISSN: 0021-9258(print)
- ADLER ANDREAS ET AL: "Improving compliance to colorectal cancer screening using blood and stool based tests in patients refusing screening colonoscopy in Germany.", BMC GASTROENTEROLOGY 2014, vol. 14, 2014, page 183, ISSN: 1471-230X
- TURNBAUGH PETER J ET AL: "An obesity-associated gut microbiome with increased capacity for energy harvest", NATURE (LONDON), vol. 444, no. 7122, December 2006 (2006-12), pages 1027-1031, ISSN: 0028-0836
- TURNBAUGH PETER J ET AL: "A core gut microbiome in obese and lean twins", NATURE (LONDON), vol. 457, no. 7228, January 2009 (2009-01) , page 480, ISSN: 0028-0836

## Description

The present invention relates to the use of the bacterial flora for vaccine development, identification of therapeutic targets and prediction and/or diagnosis of metabolic diseases, such as diabetes, overweight and obesity, and their cardiovascular complications. In particular, the present invention provides immunogenic or vaccinal composition for preventing metabolic diseases and cardiovascular complications using attenuated or inactivated bacterial flora, or antigens derived thereof. The present invention also provides methods for identifying prognostic markers for metabolic diseases onset and cardiovascular complications and methods for *in vitro* prognosis of metabolic diseases and cardiovascular complications using bacterial flora.

Metabolic diseases, such as diabetes, overweight and obesity, both in developed and emerging countries have reached epidemic proportions. The set-up of a low tonus but chronic metabolic-type inflammatory reaction is one of the cellular mechanisms that appear early during the development of these diseases. The active molecules which are released by the inflammatory process lower the insulin action and secretion of insulin or any metabolically active hormone, impair the nervous system and favour the development of fat tissue and atheroma plaques leading to cardiovascular complications.

A body of evidence demonstrates that the intestinal microbiota, which corresponds to the overall bacterial community present in the intestine, and their corresponding expressed genes, which define the microbiome, play a role in the onset of metabolic diseases such as diabetes and obesity (Turnbaugh et al., Nature, 2006, 444:1027-31 ; Turnbaugh et al., Nature, 2009, 457:480-4 ; Cani et al., Diabetes, 2007, 56:1761-72). The causal role of the intestinal microbiota on weight gain was demonstrated in experiments in which germ-free mice colonized with intestinal microbiota from genetically obese *ob*/*ob* mice gained more weight than their counterparts colonized with microbiota from lean animals (Turnbaugh et al., Nature, 2006, 444:1027-31). In humans, Turnbaugh *et al.* showed that obesity is associated with phylum-level changes in the gut microbiota and reduced bacterial diversity (Turnbaugh et al., Nature, 2009, 457:480-4). Furthermore, it has been demonstrated that gut microbiota affects energy balance by influencing the efficiency of calorie harvest from the diet and the way this harvested energy is used and stored (Turnbaugh et al., Nature, 2006, 444:1027-31). In addition, the role of bacterial components within blood in relation to weight and glucose metabolism has also been demonstrated: mice fed normally and chronically infused with a low dose of lipopolysaccharides (LPS) developed inflammation, diabetes and obesity whereas mice carrying a deletion in the gene for CD14, a component of the principal receptor for bacterial LPS, did not (Cani et al., Diabetes, 2007, 56:1761-72). Interestingly, in humans, plasma LPS concentrations are increased in apparently healthy subjects eating a high-fat diet (Amar et al., Am J Clin Nutr., 2008, 87:1219-23). Moreover, the concentration of bacterial DNA such as 16S rDNA and *LpxB* DNA in the blood of a subject has been proved to be a reliable risk marker and a predictor of metabolic and/or cardiovascular diseases (WO/2010/092164). Kalliomaki et al. American Journal of Clinical Nutrition, 2008, 87(3):534-538 discloses the follow-up of the weight of a children population with analysis of the gut microflora.

It is predicted that the number of diabetic patients will increase from 285 million adults in 1997 to about 439 million in 2025 (Shaw et al., Diabetes Res Clin Pract., 2010, 87:4-14). Therefore, it is of utmost importance to detect those at risk of metabolic diseases at an early stage, when lifestyle modifications may be efficient and easier to install. In this respect, the current recognized risk factors for diabetes, such as central adiposity or high fasting blood glucose, are markers of an advanced stage of metabolic disease. Furthermore, there is a need for markers of diabetes, overweight and obesity risk that are independent of metabolic features in order to generate new concepts for therapeutic strategies. Due to the deleterious impact of these metabolic diseases and their cardiovascular complications in human health, there is a need to develop vaccination strategies for these diseases and complications and to find parameters which allow their prognosis.

In this study, the inventors surprisingly identified that the bacterial DNA present in blood or in subcutaneous adipose tissue and more broadly in any tissue can be the basis for the development of prognostic or diagnostic biomarkers of metabolic diseases onset. The inventors observed that the presence of 16S rDNA gene concentration of a specific phylum, family or genus of bacteria could be a prognostic or diagnostic marker of diabetes, overweight, obesity and morbide obesity. On the basis of these results, they demonstrated that specifically identified phyla, families and/or genera of bacteria can be the support to vaccination strategies against metabolic diseases.

The invention is as defined in the claims.

### Definitions

In the context of the invention, a "metabolic disease" denotes a disease that disrupts normal metabolism. Preferably, the metabolic disease according to the invention is a carbohydrate metabolism disorder.

As used herein a "carbohydrate metabolism disorder" denotes a disease wherein the metabolism of carbohydrate, for example of glucose, is disrupted. Carbohydrate metabolism disorders include diabetes, high fasting glycemia, overweight, obesity and morbide obesity.

As used herein, "diabetes" denotes a syndrome of disordered metabolism, usually due to a combination of hereditary and environmental causes, resulting in a concentration of glucose in plasma superior to 7 mmol/L.

"Overweight", "obesity" and "morbide obesity" are defined as abnormal or excessive fat accumulation that may impair health. Body mass index (BMI) is a simple index of weight-for-height that is commonly used in classifying overweight and obesity in adult populations and individuals. It is defined as the weight in kilograms divided by the square of the height in meters (kg/m2). "Overweight" is defined by a BMI equal to or more than 25, "obesity" as a BMI equal to or more than 30 and "morbide obesity" as a BMI equal to or more than 40.

In the context of the invention, a "cardiovascular complication" refers to a disease that results from the onset of a metabolic disorder and affects the heart or blood vessels (arteries and veins). More particularly, a cardiovascular complication as disclosed herein denotes a disease, lesion or symptom associated with an atherogenesis process that affects the cardiovascular system. It includes especially the conditions in which an atheroma plaque develops as well as the complications due to the formation of an atheroma plaque (stenosis, ischemia) and/or due to its evolution toward an acute ischemic stroke (thrombosis, embolism, infarction, arterial rupture).

Cardiovascular complications include coronary artery disease, coronary heart disease, hypertension, atherosclerosis, in particular iliac or femoral atherosclerosis, angina pectoris, thrombosis, heart failure, stroke, vascular aneurysm, vascular calcification, acute coronary syndromes such as myocardial infarction, vascular stenosis and infarction, and vascular dementia. Preferably, the cardiovascular disease as disclosed herein is selected from the group consisting of coronary artery disease, hypertension, atherosclerosis, vascular aneurysm, vascular calcification, vascular dementia and heart failure. More preferably, the cardiovascular disease as disclosed herein is atherosclerosis. Most preferably, the cardiovascular disease as disclosed herein the invention is atherosclerotic carotid plaques.

In the context of the invention, "bacterial DNA" refers to a DNA belonging to any bacteria. In particular, a bacterial DNA according to the invention denotes a DNA sequence from the genome of a bacterium.

Preferably, the bacterial DNA is 16S rDNA. More preferably, the bacterial DNA is the V2 region of 16S rDNA.

As used herein, "16S rDNA" refers to the gene encoding the 16S ribosomal RNA (16S rRNA DNA) constituted of about 1500 nucleotides, which is the main component of the small prokaryotic ribosomal subunit (30S). 16S rDNA is highly conserved among bacteria.

As used herein, V2 region of 16S rDNA refers to an hypervariable region of the 16S rDNA that can provide species-specific signature sequences useful for bacterial identification.

As used herein, a "biological sample" denotes a biological tissue, such as an intestine tissue, or its content, an adipose tissue, liver, muscles, etc, or a body fluid such as saliva, urines, blood, plasma, serum. In a preferred embodiment, the biological sample is a sample of intestine or subcutaneous adipose tissue or vascular stroma of subcutaneous tissue or blood or plasma.

As used herein, the term "subject" denotes a non-human mammal or a human. Preferred non-human mammals include primates, rodents, such as mouse or rat, feline, canine, bovine and ovine. More particularly, the subject is a human, in particular a child, an adult, a woman or a man.

### Method for identifying markers of metabolic disease and cardiovascular complications

The specification discloses a method for identifying a phylum, family and/or genus of bacteria, which is a prognostic marker of metabolic diseases, and optionally of cardiovascular complications, which method comprises the steps consisting of:
a) quantifying bacteria in a biological sample from a subject healthy at the time of sampling and who developed metabolic diseases, and optionally of cardiovascular complications, within a ten year period after sampling;
b) comparing said quantified bacteria of step a) with the bacteria from a control; and
c) identifying phyla, families and/or genera of bacteria which are present in different proportions within the sample assayed in step a) and the control;
wherein said identified phyla, families and/or genera of bacteria which are present in different proportions are prognostic markers of metabolic diseases, and optionally of cardiovascular complications.

Preferably, as disclosed herein, the metabolic disease is selected from the group consisting of diabetes, overweight, obesity and/or morbide obesity.

The invention refers to identifying a phylum, family and/or genus of bacteria, which is a prognostic marker of diabetes, which method comprises the steps consisting of :
a) quantifying bacteria in a biological sample from a subject healthy at the time of sampling and who developed diabetes, within a ten year period after sampling;
b) comparing said quantified bacteria of step a) with the bacteria from a control; and
c) identifying phyla, families and/or genera of bacteria which are present in different proportions within the sample assayed in step a) and the control;
wherein said identified phyla, families and/or genera of bacteria which are present in different proportions are prognostic markers of diabetes, wherein the control refers to a subject healthy at the time of sampling who did not develop diabetes within a ten year period of sampling and,
wherein said biological sample of step a) and the control sample is blood.

The invention further refers to a method for identifying a phylum, family and/or genus of bacteria, which is a prognostic marker of overweight, obesity and/or morbide obesity, which method comprises the steps consisting of :
a) quantifying bacteria in a biological sample from a subject healthy at the time of sampling and who developed overweight, obesity and/or morbide obesity, within a ten year period after sampling;
b) comparing said quantified bacteria of step a) with the bacteria from a control; and
c) identifying phyla, families and/or genera of bacteria which are present in different proportions within the sample assayed in step a) and the control;
wherein said identified phyla, families and/or genera of bacteria which are present in different proportions are prognostic markers of overweight, obesity and/or morbide obesity, wherein the control refers to a subject healthy at the time of sampling who did not develop overweight, obesity and/or morbide obesity within a ten year period of sampling and,
wherein said biological sample of step a) and the control sample is adipose subcutaneous tissue, or vascular stroma of adipose subcutaneous tissue.

The bacteria quantity may be determined by quantifying the amount of bacterial DNA and more preferably the bacterial 16S rDNA gene by methods known to the one skilled in the art such as for example by quantitative polymerase chain reaction (PCR), most preferably by real-time quantitative PCR. More preferably, the V2 variable region of 16s rDNA gene is quantified by quantitative PCR.

Preferably, when the amplification is performed by PCR, a reporter that produces fluorescence, such as SYBR Green and "universal primers" referring to primers comprising a sequence which is able to hybridize to 16S rDNA from essentially any origin, are used. In a preferred embodiment, the "universal primers" according to the invention are the forward eubac-F primer of sequence 5'-TCCTACGGGAGGCAGCAGT-3' (SEQ ID NO: 1) and the reverse eubac-R primer of sequence 5'-GGACTACCAGGGTATCTAATCCTGTT-3' (SEQ ID NO: 2). In another preferred embodiment, the "universal primers" according to the invention are the forward F-Bact1369 primer of sequence 5'-CGGTGAATACGTTCCCGG-3' (SEQ ID NO: 3) and the reverse R-Prok1492 primer of sequence 5'-TACGGCTACCTTGTTACGACTT-3' (SEQ ID NO: 4).

As disclosed herein , in step a) the quantification of bacteria may be performed in a biological sample of a subject healthy at the time of sampling and who developed a metabolic disease and optionally cardiovascular complications within a ten year period after sampling, preferably within a 9, 8, 7, 6, 5, 4, 3, 2 or 1 year period after sampling.

The method according to the invention also involves comparing the quantified bacteria with the bacteria of a control. The "control" denotes a subject who was healthy at the time of sampling and who did not developed a metabolic disease within at least a 3, 6 or 9 year period after sampling and preferably at least within a ten year period of sampling.

Identification of phyla, families and/or genera which are present in different proportions within the sample assayed in step a) and the control may be performed by sequencing the V2 variable region of 16s rDNA gene after its amplification by quantitative PCR. In a preferred embodiment, the said identification of phyla, families and/or genera is performed by pyrosequencing the V2 variable region of 16s rDNA gene. In a preferred embodiment, the primers are the forward primer of sequence 5'-AGAGTTTGATCMTGGCTCAG -3' (SEQ ID NO: 5) and the reverse eubac-R primer of sequence 5'- GTCGCCTCCCGTAGGAGT -3' (SEQ ID NO: 6).

Identification of phyla, families and/or genera of bacteria which are present in different proportions within the sample assayed in step a) and the control means that the quantity of phyla, families and/or genera of bacteria assayed in step a) is significantly inferior or superior to the quantity of phyla, families and/or genera of bacteria in the control. By "significantly" is intented to denote a measurable and statistically significant difference. Alternatively, a significant difference may denote an at least 1%, 2%, 3%, 4%, 5%, 10%, 15 %, 20% difference in the proportions of bacteria of a phylum, a family or a genus between control and sample assayed.

Preferably the control sample is the same type of sample than the analyzed biological sample of step a). For example, if the biological sample of step a) is blood, the control sample is blood.

Preferably, if said metabolic disease is diabetes, then the biological sample of step a) and the control sample are blood samples or biopsy of subcutaneous adipose tissue.

Preferably, if said metabolic disease is overweight, obesity or morbide obesity, the biological sample of step a) and the control sample are biopsy of subcutaneous adipose tissue, and more preferably are samples of vascular stroma of subcutaneous adipose tissue.

### Method for in vitro prognosis of metabolic disease and cardiovascular complications

The inventors identified that apparently healthy patients, who will develop a metabolic disease, and optionally cardiovascular complications, differ from controls healthy individuals, who will not develop a metabolic disease, by the presence of a higher 16s rDNA gene concentration of specific phyla, families and/or genera of bacteria.

Thus, the specification further discloses an *in vitro* method for prognosing a metabolic disease and optionally cardiovascular complications, which method comprises the steps consisting of:
a) quantifying bacteria in a biological sample from a subject healthy at the time of sampling;
b) comparing said quantified bacteria of step a) with the bacteria from a control; and
c) identifying phyla, families and/or genera of bacteria which are prognostic markers of metabolic disease and optionally of cardiovascular complications and
which are present in different proportions within the sample of the individual assayed in step a) and the control;
wherein said presence in different proportions indicates that the subject is at risk of developing a metabolic disease and optionally cardiovascular complications.

Preferably, as disclosed herein, the metabolic disease is selected from the group consisting of diabetes, overweight, obesity and/or morbide obesity.
The invention refers to an *in vitro* method for prognosing diabetes, which method comprises the steps consisting of :
a) quantifying bacteria in a biological sample from a subject healthy at the time of sampling;
b) comparing said quantified bacteria of step a) with the bacteria from a control; and
c) identifying phyla, families and/or genera of bacteria which are prognostic markers of diabetes and which are present in different proportions within the sample of the individual assayed in step a) and the control;
wherein said presence in different proportions indicates that the subject is at risk of developing diabetes,
wherein the control refers to a subject healthy at the time of sampling who did not develop diabetes within a ten year period of sampling and,
wherein said biological sample of step a) and the control sample are blood.
The invention further refers to an *in vitro* method for prognosing overweight, obesity and/or morbide obesity, which method comprises the steps consisting of :
a) quantifying bacteria in a biological sample from a subject healthy at the time of sampling;
b) comparing said quantified bacteria of step a) with the bacteria from a control; and
c) identifying phyla, families and/or genera of bacteria which are prognostic markers of overweight, obesity and/or morbide obesity and which are present in different proportions within the sample of the individual assayed in step a) and the control;
wherein said presence in different proportions indicates that the subject is at risk of developing overweight, obesity and/or morbide obesity,
wherein the control refers to a subject healthy at the time of sampling who did not develop overweight, obesity and/or morbide obesity within a ten year period of sampling and,
wherein said biological sample of step a) and the control sample is adipose subcutaneous tissue, or vascular stroma of adipose subcutaneous tissue.
**6.** The method according to claim 5, wherein the identification of bacteria of the Proteobacteria phylum in step c) indicates that the subject is at risk of developing overweight, obesity and/or morbide obesity.

The bacteria quantity may be determined by quantifying the amount of bacterial DNA and more preferably the bacterial 16S rDNA gene by methods known to the one skilled in the art such as for example by quantitative PCR, most preferably by real-time quantitative PCR. More preferably, the V2 variable region of 16s rDNA gene is quantified.

According to the method of the invention, in step a) the quantification of bacteria is performed in a biological sample of a subject healthy at the time of sampling.

The method according to the invention also involves comparing the quantified bacteria with the bacteria of a control. The "control" denotes a subject who was healthy at the time of sampling and who did not developed a metabolic disease within at least a 3, 6 or 9 year period after sampling and preferably at least within a ten year period of sampling.

Identification of phyla, families and/or genera which are prognostic markers and which are present in different proportions within the sample assayed in step a) and the control may be performed by sequencing the V2 variable region of 16s rDNA gene after its amplification by PCR.

Preferably said phyla of bacteria which are prognostic markers of metabolic disease onset and optionally of cardiovascular complications are prognostic markers of diabetes, overweight, obesity and/or morbide obesity. Preferably said phyla of bacteria which are prognostic markers of diabetes, overweight, obesity and/or morbide obesity are selected from the group consisting of Proteobacteria and Firmicutes. More preferably, the Proteobacteria phylum is a prognostic marker of diabetes onset, overweight onset, obesity onset and/or morbide obesity onset and the Firmicutes phylum is a prognostic marker of overweight onset, obesity onset and/or morbide obesity onset. Preferably said families of bacteria which are prognostic markers of metabolic disease and optionally of cardiovascular complications are prognostic markers of diabetes, overweight, obesity and/or morbide obesity. Preferably said families of bacteria which are prognostic markers of diabetes are any of families of the Proteobacteria phylum, and said families of bacteria which are prognostic markers of overweight, obesity and/or morbide obesity are any of the families of the Proteobacteria phylum and/or the Firmicutes phylum. More preferably, said families which are prognostic markers of diabetes, overweight, obesity and/or morbide obesity are selected from the group consisting of *Burkhloderiaceae, Pseudomonaceae, Oxalobacteriaceae, Neisseriaceae, Enterobacteriaceae, Moraxellaceae, Streptococcaceae, Lactobacillaceae, Staphylococcaceae, Leuconostocaceae, Clostridiaceae* and *Veillonellaceae.* More preferably, *Burkhloderiaceae* and/or *Pseudomonaceae* families of the Proteobacteria phylum are prognostic markers of diabetes, and *Oxalobacteriaceae, Burkhloderiaceae, Neisseriaceae, Enterobacteriaceae* and/or *Moraxellaceae* families of the Proteobacteria phylum and/or *Lactobacillaceae, Leuconostocaceae, Clostridiaceae* and/or *Veillonellaceae* families of the Firmicutes phylum are prognostic markers of overweight, and *Oxalobacteriaceae, Burkhloderiaceae, Neisseriaceae, Enterobacteriaceae* and/or *Moraxellaceae* families of the Proteobacteria phylum and/or *Streptococcaceae, Lactobacillaceae, Clostridiaceae, Veillonellaceae* and/or *Staphylococcaceae* families of the Firmicutes phylum are prognostic markers of obesity and/or morbide obesity.

Preferably said genera of bacteria which are prognostic markers of metabolic disease and optionally of cardiovascular complications are prognostic markers of diabetes, overweight, obesity and/or morbide obesity. Preferably said genera of bacteria which are prognostic markers of diabetes are any of the genera of the Proteobacteria phylum and said genera of bacteria which are prognostic markers of overweight, obesity and/or morbide obesity are any of genera of the Proteobacteria phylum and/or the Firmicutes phylum. More preferably, said genera of bacteria which are prognostic markers of diabetes, overweight, obesity and/or morbide obesity are selected from the group consisting of *Ralstonia, Undibacterium, Neisseriales, Enteric Bacteria Cluster, Enhydrobacter* and/or *Acinetobactergenera* of the Proteobacteria phylum.

Identification of said phyla and/or genera which are present in different proportions within the sample assayed in step a) and the control means that the quantity of phyla and/or genera of bacteria assayed in step a) is significantly inferior or superior than the quantity of phyla and/or genera of bacteria in the control. By "significantly" is intented to denote a measurable and statistically significant difference. Alternatively, a significant difference may denote an at least 1%, 2%, 3%, 4%, 5%, 10%, 15 %, 20% difference in the proportions of bacteria of a phylum, a family or a genus between control and sample assayed.

Preferably the control sample is the same type of sample than the analyzed biological sample of step a). For example, if the biological sample of step a) is blood, the control sample is blood.

Preferably, if said metabolic disease is diabetes, then the biological sample of step a) and the control sample are blood samples.

Preferably, if said metabolic disease is overweight or obesity, the biological sample of step a) and the control sample are biopsy of adipose tissue, and more preferably are samples of vascular stroma of adipose tissue.

### Compositions and therapeutic applications

The inventors demonstrated that administration of a composition comprising an antigen preparation of attenuated and/or inactivated bacteria of at least one phylum, family and/or genus of bacteria which is a prognostic marker of metabolic disease and optionally of cardiovascular complications as defined above is capable of eliciting an immune response protecting an individual against metabolic disease and cardiovascular complications.

Thus the specification discloses an immunogenic or vaccinal composition comprising or consisting of:
a) attenuated or inactivated bacteria of at least one phylum, family and/or genus of bacteria which is a prognostic marker of metabolic disease and optionally of cardiovascular complications; or
b) at least one immunogenic peptide from a bacterium of a phylum, family and/or genus of bacteria which is a prognostic marker of metabolic disease and optionally of cardiovascular complications.

The specification also discloses attenuated or inactivated bacteria of at least one phylum, family and/or genus of bacteria which is a prognostic marker of metabolic disease and optionally of cardiovascular complications, or at least one immunogenic peptide from a bacterium of a phylum, family and/or genus of bacteria which is a prognostic marker of metabolic disease and optionally of cardiovascular complications, for use for preventing and/or treating metabolic disease and optionally of cardiovascular complications.

As disclosed herein, attenuated or inactivated bacteria may originate from a biological tissue such as an adipose tissue, liver, muscles, or from a body fluid such as saliva, urines, blood, plasma, serum. Preferably, attenuated or inactivated bacteria do not originate from the intestine tissue content.

By "originate" it is meant that, e.g. attenuated or inactivated bacteria originating from blood were obtained by attenuating or inactivating bacteria isolated from a blood sample.

In the context of the invention, the term "treating" or "treatment", as used herein, means reversing, alleviating, inhibiting the progress of, or preventing the disorder or condition to which such term applies, or one or more symptoms of such disorder or condition.

Preferably, the metabolic disease is selected from the group consisting of diabetes, overweight, obesity and/or morbide obesity.

According to particular embodiments of the present invention, said at least one phylum, family and/or genus of bacteria which is a prognostic marker of metabolic disease onset and optionally of cardiovascular complications onset is selected from the group consisting of Proteobacteria and Firmicutes phyla.

Preferably, said family of bacteria which is a prognostic marker of metabolic disease onset and optionally cardiovascular complications onset is selected from the group consisting of *Burkhloderiaceae, Pseudomonaceae, Oxalobacteriaceae, Neisseriaceae, Enterobacteriaceae, Moraxellaceae, Streptococcaceae, Lactobacillaceae, Staphylococcaceae, Leuconostocaceae, Clostridiaceae* and *Veillonellaceae.*

Preferably, said genus of bacteria which is a prognostic marker of metabolic disease onset and optionally cardiovascular complications onset is selected from the group consisting of *Ralstonia, Undibacterium, Neisseriales, Enteric Bacteria Cluster, Enhydrobacter* and/or *Acinetobacter.*

In particular preferred embodiments, said phylum which is a prognostic marker of diabetes, overweight, obesity and/or morbide obesity consists of the Proteobacteria phylum, and said phylum which is a prognostic marker of overweight, obesity and/or morbide obesity consists of the Firmicutes phylum.

In still particular embodiments, said family which is a prognostic marker of diabetes is selected from the group consisting of *Burkhloderiaceae, Pseudomonaceae* families of Proteobacteria phylum, family which is a prognostic marker of overweight is selected from the group consisting of *Burkhloderiaceae, Oxalobacteriaceae, Neisseriaceae, Enterobacteriaceae* and *Moraxellaceae* families of the Proteobacteria phylum and *Lactobacillaceae, Leuconostocaceae, Clostridiaceae* and *Veillonellaceae* families of the Firmicutes phylum, and family which is a prognostic marker of obesity and/or morbide obesity is selected from the group consisting of *Burkhloderiaceae, Oxalobacteriaceae, Neisseriaceae, Enterobacteriaceae* and *Moraxellaceae* families of the Proteobacteria phylum and *Streptococcaceae, Lactobacillaceae, Staphylococcaceae, Clostridiaceae* and *Veillonellaceae* of the Firmicutes phylum.

In still particular embodiments, said genus which is a prognostic marker of diabetes, overweight, obesity and/or morbide obesity is selected from the group consisting of *Ralstonia, Undibacterium, Neisseriales, Enteric Bacteria Cluster, Enhydrobacter* and/or *Acinetobactergenera* of the Proteobacteria phylum.

As used herein, an "attenuated bacteria" is a live bacterium whose aggressiveness against the body is weak or null.

An "inactivated bacteria" refers to whole-killed bacteria.

Methods of obtaining attenuated or inactivated bacteria are well known in the art.

As used herein, an "immunogenic peptide" is a protein or a fragment thereof obtained from a bacterium. Preferably, the immunogenic peptide is a flagellae protein, a pili protein, a secreted protein, an internal protein or a fragment thereof.

Methods of obtaining immunogenic peptide are well known in the art.

"Immunogenic composition" as disclosed herein relates to a composition comprising at least one protein or fragment thereof (hereafter referred to as "antigen") which provokes or immunomodulates (*i.e.* immunosuppress or immunostimulate) an immune response when administered to an individual. Preferably, the protein which provokes or immunomodulates an immune response is a flagellae protein, a pili protein, a secreted protein, an internal protein or a fragment thereof.

A "vaccinal composition" as used herein refers to a composition, such as the immunogenic composition described herein which is administered to immunomodulate an immune response, that will protect or treat an individual from illness, in particular due to that agent. The vaccine as disclosed herein is intended for use both as a therapeutic (treatment) vaccine, *i.e.* for administration to the individual after development of the disease with the intention to reduce or arrest disease progression and as a preventive (prophylactic) vaccine, for administration to the individual prior to the development of the disease, with the intent to prevent initial (and/or recurrent) infection.

For use as a vaccine as disclosed herein the immunogenic composition may comprise whole-killed (inactive) bacteria, live-attenuated bacteria or processed and/or artificial bacterial preparations or combinations thereof. Processed bacterial preparations included preparations of bacterial proteins which are partially or completely purified and/or pretreated.

Methods of obtaining immunogenic composition are well known in the art. Generally such methods involve extracting proteins from bacterial preparations using techniques such as sonication, proteolytic digestion, heat treatment, freeze-thaw treatment, osmotic shock treatment etc.... Examples of artificial bacterial preparations include protein preparations either in part or entirely obtained by synthetic or recombinant methods. Oligonucleotides and probes can be devised based on the sequences of the bacterial genome and can be used to probe genomic or cDNA libraries for genes encoding antigens useful in the context of the invention. Genes can be isolated using standard techniques.

The object of the immunogenic or vaccinal composition as disclosed herein includes obtaining at least partial and preferably complete protection against diabetes, overweight, obesity and/or morbide obesity by preventing the apparition or development of at least one phylum, family and/or genus of bacteria which is a prognostic marker of diabetes, overweight , obesity and/or morbide obesity onset, and/or by reducing the bacterial burden of at least one phylum, family and/or genus of bacteria which is a prognostic marker of diabetes, overweight, obesity and/or morbide obesity onset. Most particularly, the present specification discloses immunogenic or vaccinal composition which ensures a protective effect or reduced bacterial burden for a prolonged period of time, such as during at least 6 months, 1 years, 5 years, 10 years.

The immunogenic or vaccinal composition can comprise a suitable adjuvant. The type of adjuvant will vary, depending on the type of antigen preparation and route of administration used. Any adjuvant known in the art may be used in the immunogenic or vaccinal composition, including oil-based adjuvants such as Freund's Complete Adjuvant and Freund's Incomplete Adjuvant, mycolate-based adjuvants (e.g., trehalose dimycolate), bacterial lipopolysaccharides (LPS), peptidoglycans (*i.e*., mureins, mucopeptides, or glycoproteins such as N-Opaca, muramyl dipeptide [MDP], or MDP analogs), proteoglycans (e.g., extracted from Klebsiella pneumoniae), streptococcal preparations (e.g., OK432), Biostim.TM. (e.g., 01 K2), the "Iscoms" of EP 109 942, EP 180 564 and EP 231 039, aluminum hydroxide, saponin, DEAE-dextran, neutral oils (such as miglyol), vegetable oils (such as arachis oil), liposomes, Pluronic.RTM. polyols. Adjuvants include, but are not limited to, the RIBI adjuvant system (Ribi Inc.), alum, aluminum hydroxide gel, cholesterol, oil-in water emulsions, water-in-oil emulsions such as, e.g., Freund's complete and incomplete adjuvants, Block co-polymer (CytRx, Atlanta Ga.), SAF-M (Chiron, Emeryville Calif.), AMPHIGEN.RTM. adjuvant, saponin, Quil A, QS-21 (Cambridge Biotech Inc., Cambridge Mass.), GPI-0100 (Galenica Pharmaceuticals, Inc., Birmingham, Ala.) or other saponin fractions, monophosphoryl lipid A, Avridine lipid-amine adjuvant, heat-labile enterotoxin from E. coli (recombinant or otherwise), cholera toxin, or muramyl dipeptide, among many others.

The immunogenic or vaccinal composition disclosed herein can be administered via any suitable route, such as by mucosal (intranasal), parenteral, or intramuscular administration, oral, intradermal, intraperitoneal, intravenous, or subcutaneous administration. Suitable vaccination routes also comprise combination administrations (*e.g*. oral/intramuscular administration).

The immunogenic or vaccinal composition disclosed herein can be administered alone or with suitable pharmaceutical carriers, and can be in solid or liquid form such as, tablets, capsules, powders, solutions, suspensions, or emulsions.

The solid unit dosage forms can be of the conventional type. The solid form can be a capsule, such as an ordinary gelatin type containing the proteins or peptides disclosed herein or the antibodies or binding portions thereof disclosed herein and a carrier, for example, lubricants and inert fillers such as, lactose, sucrose, or cornstarch. In another embodiment, these compounds are tableted with conventional tablet bases such as lactose, sucrose, or corn starch in combination with binders like acacia, corn starch, or gelatin, disintegrating agents such as, corn starch, potato starch, or alginic acid, and a lubricant like stearic acid or magnesium stearate.

The immunogenic or vaccinal composition disclosed herein may also be administered in injectable dosages by solution or suspension of these materials in a physiologically acceptable diluent with a pharmaceutical carrier. Such carriers include sterile liquids such as water and oils, with or without the addition of a surfactant and other pharmaceutically acceptable adjuvants. Illustrative oils are those of petroleum, animal, vegetable, or synthetic origin, for example, peanut oil, soybean oil, or mineral oil. In general, water, saline, aqueous dextrose and related sugar solution, and glycols such as, propylene glycol or polyethylene glycol, are preferred liquid carriers, particularly for injectable solutions.

For use as aerosols, the immunogenic or vaccinal composition disclosed herein in solution or suspension may be packaged in a pressurized aerosol container together with suitable propellants, for example, hydrocarbon propellants like propane, butane, or isobutane with conventional adjuvants. The materials disclosed herein also may be administered in a non-pressurized form such as in a nebulizer or atomizer.

Is also disclosed herein a method of immunizing and/or vaccinating a patient against metabolic disease and optionally of cardiovascular complications, wherein said method comprises the administration to the patient of an immunogenic or vaccinal composition comprising or consisting of:
a) attenuated or inactivated bacteria of at least one phylum, family and/or genus of bacteria which is a prognostic marker of metabolic disease and optionally of cardiovascular complications; or
b) at least one immunogenic peptide from a bacterium of a phylum, family and/or genus of bacteria which is a prognostic marker of metabolic disease and optionally of cardiovascular complications.

### FIGURES

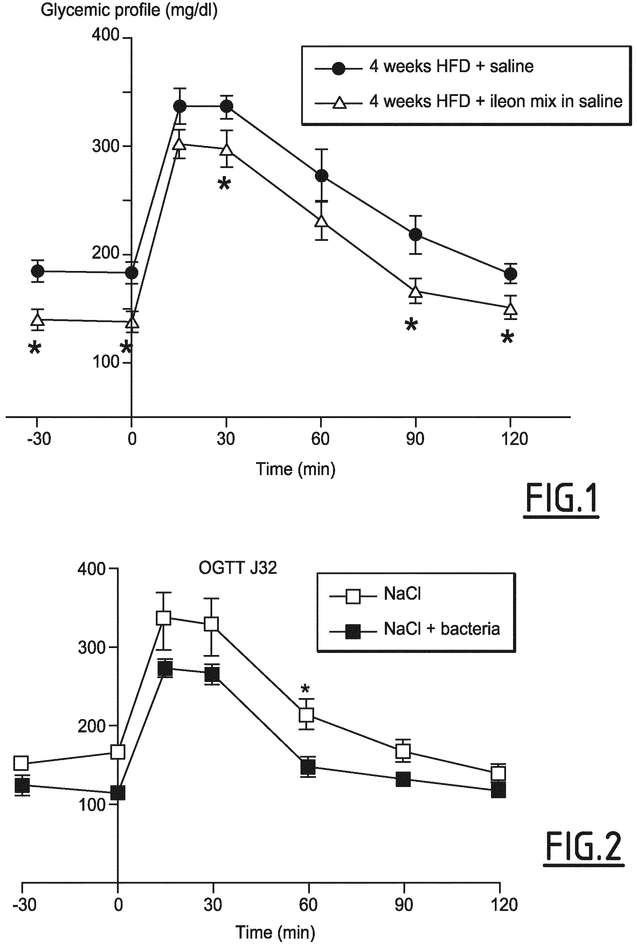

**Figure 1** depicts glycemic profiles of 9 weeks-old C57BI6J male mice which were subcutaneously injected with ultrasonically inactivated bacteria issued from intestinal microbiota (ileum mix) originating from mice normally fed and diluted in sterile saline or with saline alone. 3 injections were performed 10 days before, 3 days before and 4 days after the beginning of hight fat diet (HFD). Mean ± sem, n=12. * : p<0.05 versus 4 weeks HFD + saline. An oral glucose tolerance test (1g/kg) has been performed one month after the beginning of the HFD period. * significantly different from saline injected mice when p<0,05.
**Figure 2** depicts glycemic profiles 32 days after the last injection of 9 weeks-old C57BI6J male mice which were subcutaneously injected with ultrasonically inactivated intestinal bacteria (ileum mix) diluted in sterile saline. 3 injections were performed 10 days before, 3 days before and 4 days after the beginning of hight fat diet (HFD).

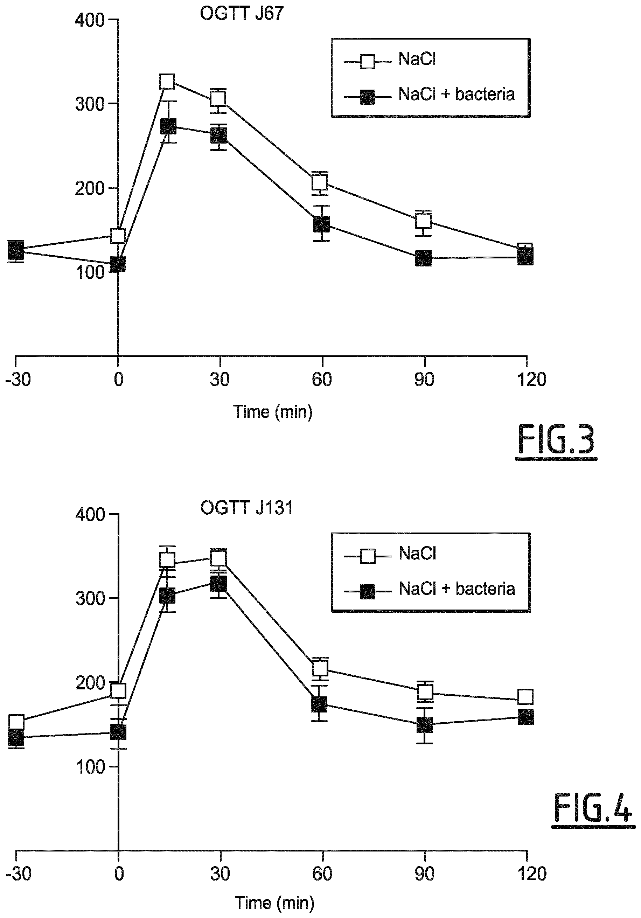

**Figure 3** depicts glycemic profiles 67 days after the last injection of 9 weeks-old C57BI6J male mice which were subcutaneously injected with ultrasonically inactivated intestinal bacteria (ileum mix) diluted in sterile saline. 3 injections were performed 10 days before, 3 days before and 4 days after the beginning of hight fat diet (HFD).
**Figure 4** depicts glycemic profiles 131 days after the last injection of 9 weeks-old C57BI6J male mice which were subcutaneously injected with ultrasonically inactivated intestinal bacteria (ileum mix) diluted in sterile saline. 3 injections were performed 10 days before, 3 days before and 4 days after the beginning of hight fat diet (HFD).

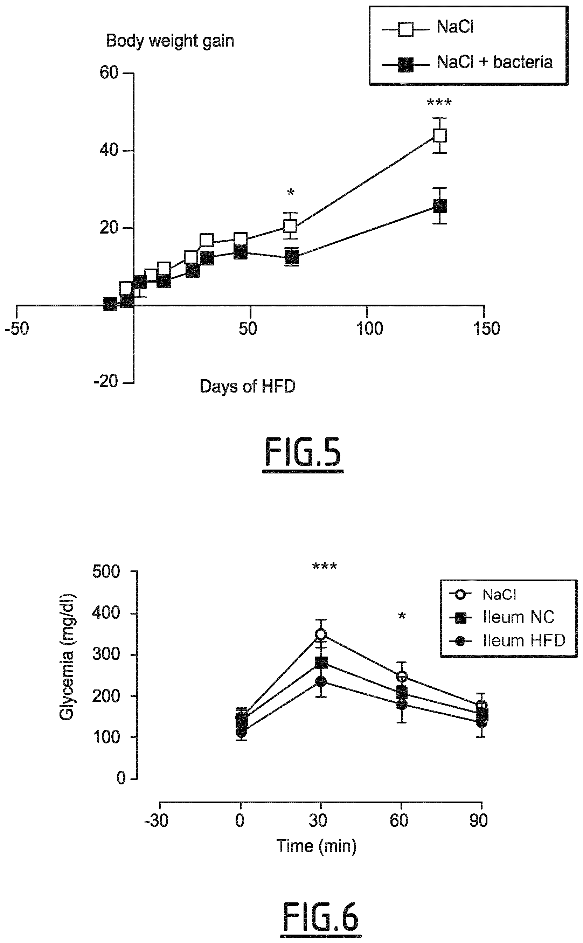

**Figure 5** depicts the body weight gain profiles of 9 weeks-old C57BI6J male mice which were subcutaneously injected with ultrasonically inactivated intestinal bacteria (ileum mix) diluted in sterile saline. 3 injections were performed 10 days before, 3 days before and 4 days after the beginning of hight fat diet (HFD). Mean ± sem, n=12. * : p<0.05 versus 4 weeks HFD + saline.
**Figure 6** depicts glycemic profiles of C57BI6J male mice which were subcutaneously injected, when 9 weeks-old, with ultrasonically inactivated bacteria issued from intestinal microbiota (ileum NC) originating from mice normally fed (ileum NC) or rendered diabetic by a high fat diet (ileum HFD) and diluted in sterile saline or with saline alone (NaCl). 2 injections were performed 30 days and 1 day before the beginning of high fat diet (HFD). Mean ± sem, n=10. * : p<0.05 versus ileum NC and *** p<0.01 versus saline injected mice.

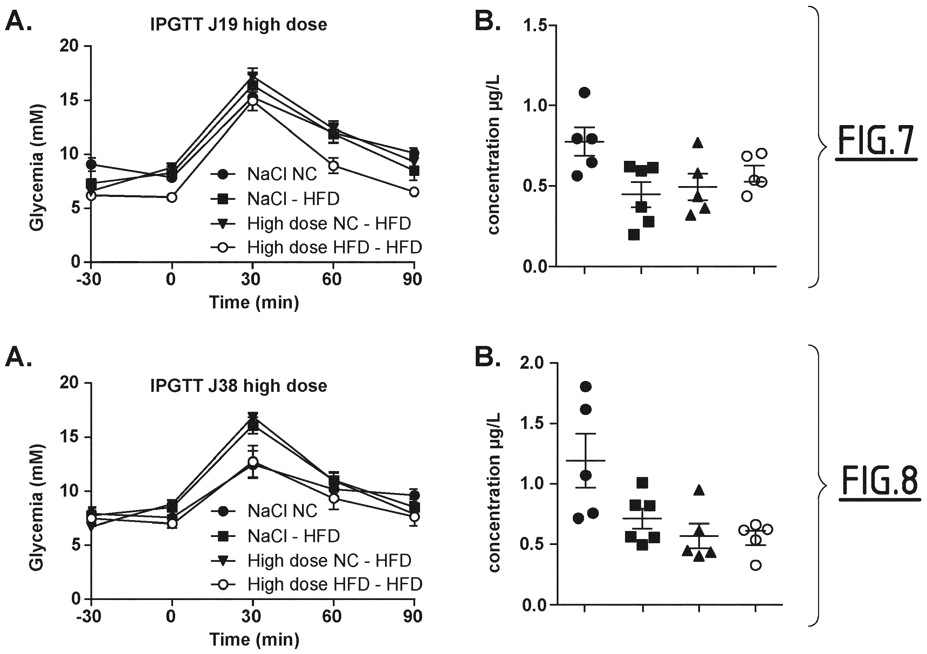

**Figure 7** depicts intraperitoneal glucose tolerance test. **A.** Glycemic profile of mice normally fed which were subcutaneously injected with NaCl (NaCl NC), mice rendered diabetic by a high fat diet which were subcutaneously injected with NaCl (NaCl HFD) 35 days prior to the diet treatment, mice rendered diabetic by a high fat diet which were subcutaneously injected with non vaccinating bacteria (High dose NC-HFD) 35 days prior to the diet treatment, and mice rendered diabetic by a high fat diet which were subcutaneously injected with vaccinating bacteria (High dose HFD-HFD) 35 days prior to the diet treatment. Mice were studied 19 days after the beginning of the diet. **B.** Area under curve of the glycemic profiles. Mice were studied 19 days after the beginning of the diet.
**Figure 8** depicts intraperitoneal glucose tolerance test. **A.** Glycemic profile of mice normally fed which were subcutaneously injected with NaCl (NaCl NC), mice rendered diabetic by a high fat diet which were subcutaneously injected with NaCl (NaCl HFD) 35 days prior to the diet treatment, mice rendered diabetic by a high fat diet which were subcutaneously injected with non vaccinating bacteria (High dose NC-HFD) 35 days prior to the diet treatment, and mice rendered diabetic by a high fat diet which were subcutaneously injected with vaccinating bacteria (High dose HFD-HFD) 35 days prior to the diet treatment. Mice were studied 38 days after the beginning of the diet. **B.** Area under curve of the glycemic profiles. Mice were studied 38 days after the beginning of the diet.

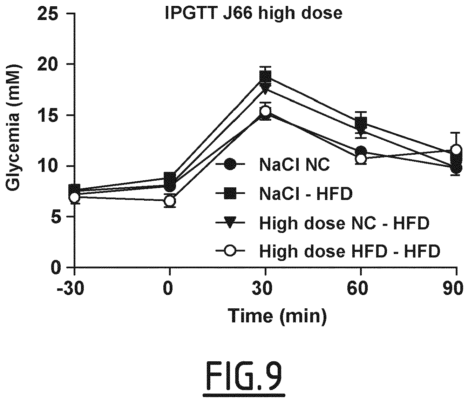

**Figure 9** depicts intraperitoneal glucose tolerance test. Glycemic profile of mice normally fed which were subcutaneously injected with NaCl (NaCl NC), mice rendered diabetic by a high fat diet which were subcutaneously injected with NaCl (NaCl HFD) 35 days prior to the diet treatment, mice rendered diabetic by a high fat diet which were subcutaneously injected with non vaccinating bacteria (High dose NC-HFD) 35 days prior to the diet treatment, and mice rendered diabetic by a high fat diet which were subcutaneously injected with vaccinating bacteria (High dose HFD-HFD) 35 days prior to the diet treatment. Mice were studied 66 days after the beginning of the diet.

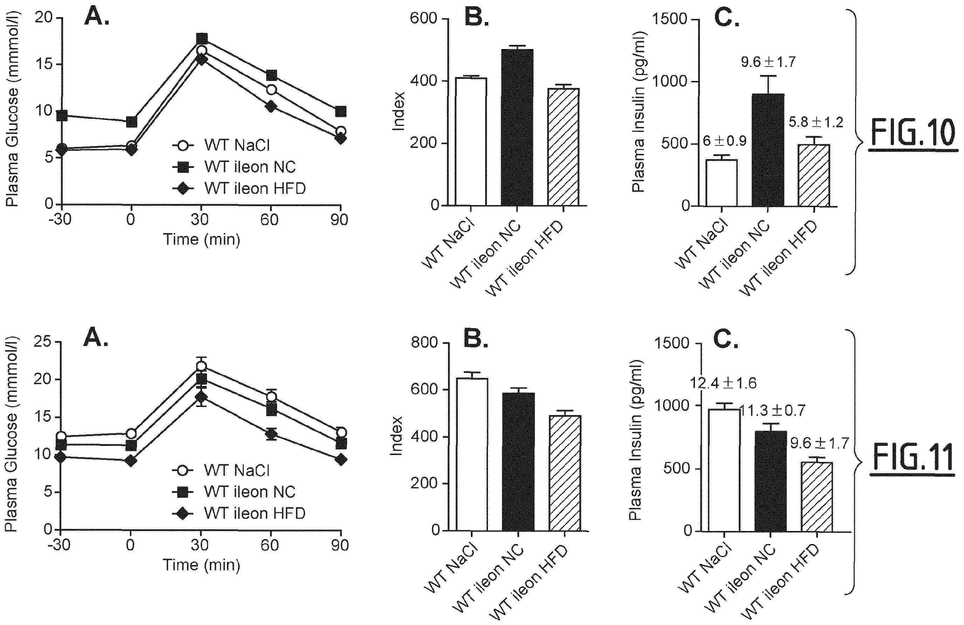

**Figure 10** depicts intraperitoneal glucose tolerance test 19 days after the beginning of the diet-induced diabetes treatment (high fat diet). **A.** Glycemic profiles of mice which were subcutaneously injected, with ultrasonically inactivated bacteria issued from intestinal microbiota originating from mice normally fed (WT ileon NC) or rendered diabetic by a high fat diet (WT ileon HFD) and diluted in sterile saline, or with saline alone (WT NaCl). **B.** Tolerance index of glucose of the mice. **C.** Insuline dosage of the mice.
**Figure 11** depicts intraperitoneal glucose tolerance test 30 days after the beginning of the diet-induced diabetes treatment (high fart diet). **A.** Glycemic profiles of mice which were subcutaneously injected, with ultrasonically inactivated bacteria issued from intestinal microbiota originating from mice normally fed (WT ileon NC) or rendered diabetic by a high fat diet (WT ileon HFD) and diluted in sterile saline, or with saline alone (WT NaCl). **B.** Tolerance index of glucose of the mice. **C.** Insuline dosage of the mice.

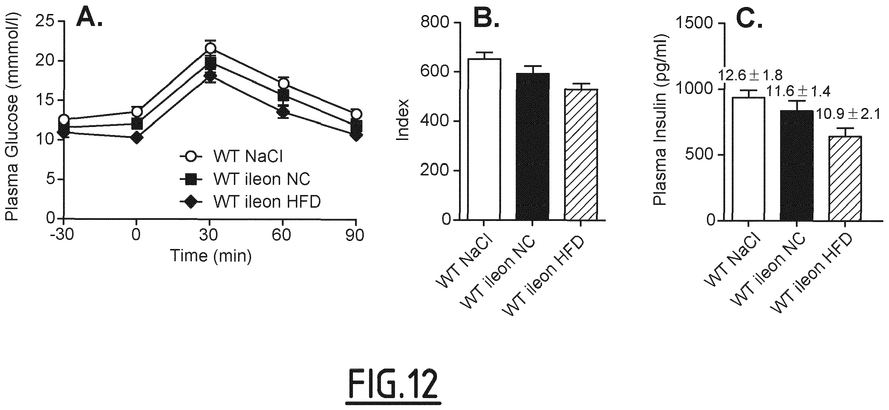

**Figure 12** depicts intraperitoneal glucose tolerance test 51 days after the beginning of the diet-induced diabetes treatment (high fart diet). **A.** Glycemic profiles of mice which were subcutaneously injected, with ultrasonically inactivated bacteria issued from intestinal microbiota originating from mice normally fed (WT ileon NC) or rendered diabetic by a high fat diet (WT ileon HFD) and diluted in sterile saline, or with saline alone (WT NaCl). **B.** Tolerance index of glucose of the mice. **C.** Insuline dosage of the mice.

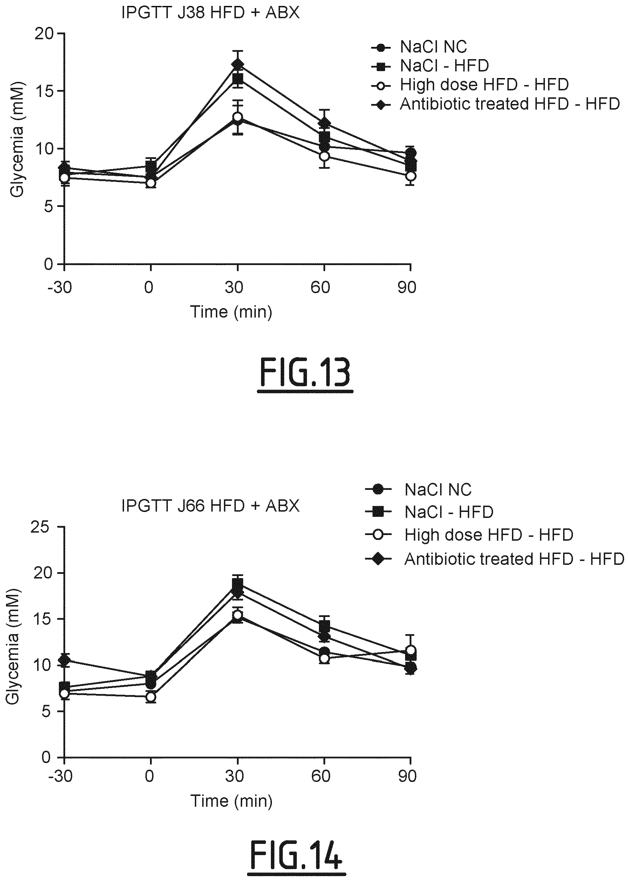

**Figure 13** depicts intraperitoneal glucose tolerance test 38 days after the beginning of the diet-induced diabetes treatment (high fart diet). Glycemic profile of mice normally fed which were subcutaneously injected with NaCl (NaCl NC), mice rendered diabetic by a high fat diet which were subcutaneously injected with NaCl (NaCl HFD), mice rendered diabetic by a high fat diet which were subcutaneously injected with vaccinating bacteria (High dose HFD-HFD), and mice rendered diabetic by a high fat diet which were subcutaneously injected with vaccinating bacteria that originates from mice treated with antibiotics to inactivate the vaccinating bacteria (Antibiotic treated HFD-HFD).
**Figure 14** depicts intraperitoneal glucose tolerance test 66 days after injection. Glycemic profile of mice normally fed which were subcutaneously injected with NaCl (NaCl NC), mice rendered diabetic by a high fat diet which were subcutaneously injected with NaCl (NaCl HFD), mice rendered diabetic by a high fat diet which were subcutaneously injected with vaccinating bacteria (High dose HFD-HFD), and mice rendered diabetic by a high fat diet which were subcutaneously injected with vaccinating bacteria that originates from mice treated with antibiotics to inactivate the vaccinating bacteria (Antibiotic treated HFD-HFD).

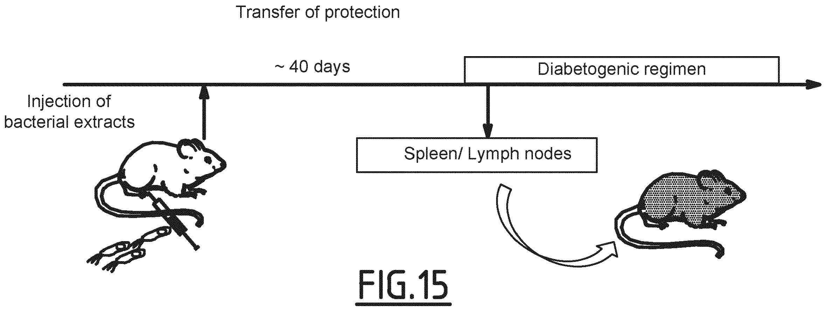

**Figure 15** depicts the protocol of transfer of protection. To demonstrate that the pretreatment of mice with the flora of the ileum coming from diabetic mice induced a vaccination by recruitment of immune cells (lymphocytes), mice are immunized and their spleen is removed and the splenic cells are transferred to another mouse. The latter is subjected to a diabetogenic diet.

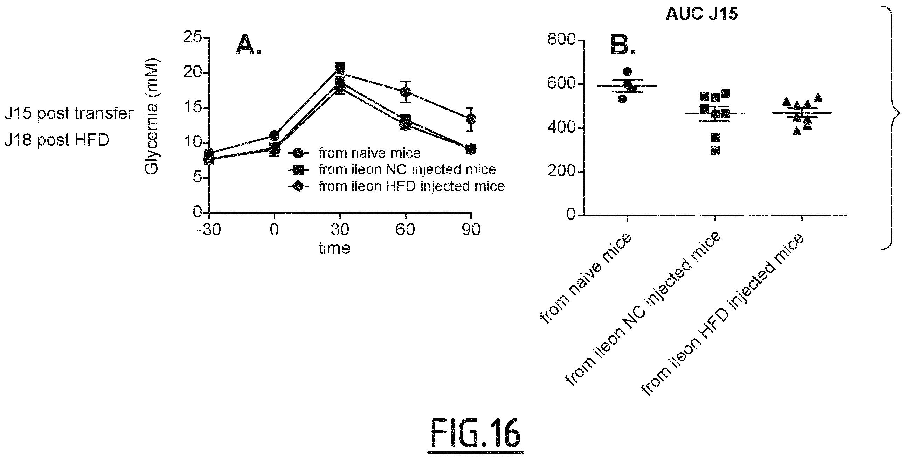

**Figure 16** depicts intraperitoneal glucose tolerance test 15 days after splenocytes transfer and 18 days after HFD. **A.** Glycemic profile of mice in which cells from spleen of non diabetic mice immunized with ileon bacterial extract (Ileon NC injected mice) or of diabetic mice immunized with ileon bacterial extract (Ileon HFD injected mice) or of naive mice has been transferred. **B.** Area under curve of the glycemic profiles.

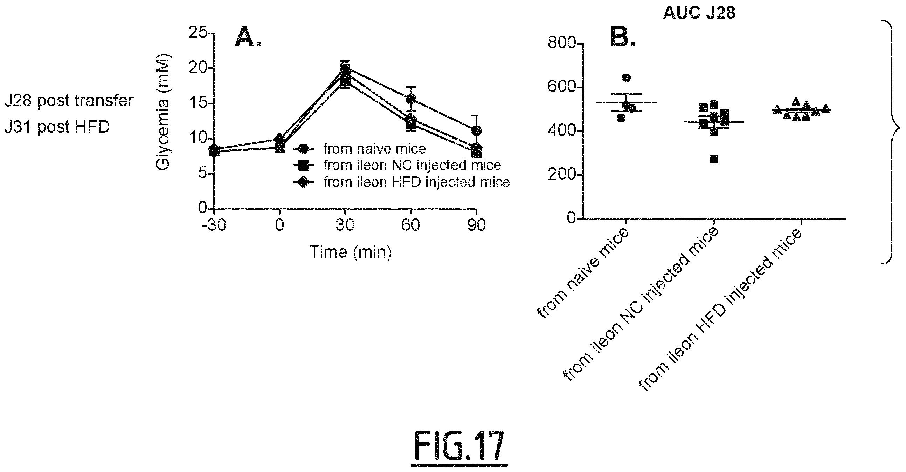

**Figure 17** depicts intraperitoneal glucose tolerance test 28 days after transfer and 31 days after HFD. **A.** Glycemic profile of mice in which spleen of non diabetic mice immunized with ileon bacterial extract (Ileon NC injected mice) or of diabetic mice immunized with ileon bacterial extract (Ileon HFD injected mice) or of maive mice has been transferred. **B.** Area under curve of the glycemic profiles.

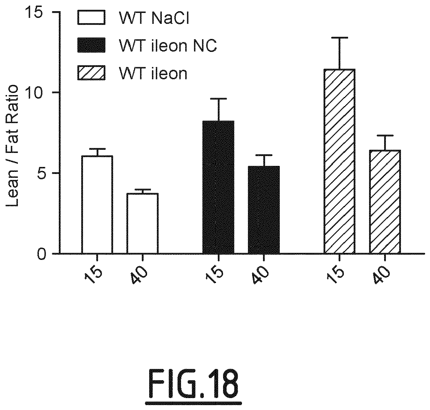

**Figure 18** depicts the lean mass / fat mass ratio of mice which were subcutaneously injected, with ultrasonically inactivated bacteria issued from intestinal microbiota originating from mice normally fed (WT ileon NC) or rendered diabetic by a high fat diet (WT ileon) and diluted in sterile saline, or with saline alone (WT NaCl).

### EXAMPLES

### Example 1: Identification of blood bacteria phyla and genera associated with diabetes.

### MATERIAL AND METHODS

### Population

D.E.S.I.R. is a longitudinal cohort study of 5,212 adults aged 30-65 years at baseline; the primary aim of the study was to describe the natural history of the metabolic syndrome (Fumeron et al., Diabetes, 2004, 53:1150-7).

Participants were recruited in 1994-1996 from ten Social Security Health Examination centers in western-central France, from volunteers insured by the French national social security system (80% of the French population - any employed or retired person and their dependents are offered free periodic health examinations). Equal numbers of men and women were recruited in five-year age groups. All participants gave written informed consent, and the study protocol was approved by the CCPPRB (Comite Consultatif de Protection des Personnes pour la Recherche Biomedicale) of Hôpital Bicêtre (Paris, France). Participants were clinically and biologically evaluated at inclusion and at 3-, 6-, and 9-year follow-up visits. We included in the analyses, those individuals who had known diabetes status at the 9-year examination, and we excluded those with C reactive protein > 30 mg/l.

Among this population, we conducted a nested control study. The study included 14 subjects at low risk of diabetes according to traditional risk factors (at baseline, these subjects were non smokers and had a waist perimeter below the median) who developed the disease after 6 years follow-up. They were matched on age, sex, central adiposity and fasting blood glucose with 25 controls free of diabetes over the entire follow-up period.

### Outcome

Incident cases of diabetes were identified by treatment for diabetes or a fasting plasma glucose ≥7.0 mmol/l at one of the four 3-yearly examinations.

### 16S rDNA gene quantification and sequencing

Total DNA concentration was determined using the Quant-iT^{™} dsDNA Broad-Range Assay Kit (Invitrogen) and a procedure adapted by the genomic platform of the Genopole Toulouse Midi Pyrenees (http://genomique.genotoul.fr). The mean concentration was 121.1 +/-2.9 ng/µl. Each sample was ten times diluted in Tris buffer EDTA. The DNA was amplified by realtime PCR (Stepone+; Applied Biosystems) in optical grade 96-well plates. The PCR reaction was performed in a total volume of 25µL using the Power SYBR® Green PCR master mix (Applied Biosystems), containing 300nM of each of the universal forward and reverse primers eubac-F 5'-TCCTACGGGAGGCAGCAGT-3' (SEQ ID NO: 1) and eubac-R 5'-GGACTACCAGGGTATCTAATCCTGTT-3' (SEQ ID NO: 2). The reaction conditions for amplification of DNA were 95°C for 10 min and 35 cycles of 95°C for 15 s and 60°C for 1 min. The amplification step was followed by a melting curve step according to the manufacturer's instructions (from 60°C to 90°C) to determine the specificity of the amplification product obtained.

Two DNA samples, extracted from blood of cases and controls, were pyrosequenced using 16S rRNA genes amplicons in order to assess their microbial composition. Following V1-V2 region amplification of the *rrs* gene by PCR, 16S rRNA amplicons were sequenced from both forward and reverse sides on a multiplexed GS FLX-Ti pyrosequencing run according to the manufacturer's protocols (Roche Applied Science, Indianapolis, IN). Sequences were binned for pyrosequencing MIDs and PCR primers removal (forward: AGAGTTTGATCMTGGCTCAG (SEQ ID NO: 5), reverse: GTCGCCTCCCGTAGGAGT (SEQ ID NO: 6) a minimal sequence length of 250 bases and a maximal threshold percentage of "N" base per sequence of 5%. Meta_RNA, a software designed for the identification of 16S rDNA gene sequences based on hidden Markov models (HMMs), was further applied to select prokaryotic *rrs* gene sequences (Huang et al., Bioinformatics, 2009, 25:1338-40). Resulting 16S rDNA sequences were assigned to phylum and genus level using RDP classifier (Cole et al., Nucleic Acids Res., 2009, 37:D141-145).

### RESULTS

### Identification of blood bacteria phyla and genera associated with diabetes

The 16S rDNA gene was sequenced in the pooled DNA sample from cases of incident diabetes and from controls without diabetes over the entire follow-up period. We first analyzed the bacterial diversity which corresponds to groups of sequences by characterizing that two sequences are different if less than 3, 5, ou 10% of their sequence differs from each other. Each group of similar sequences defines an OTU. The number of OTUs at (i) 3% was 2010 and 2179 in cases and controls, respectively, (ii) 5% was 1343 and 1493 in cases and controls, respectively and (iii) 10% was 745 and 800 in cases and controls, respectively. A common core of 443 OTUs was identified. In addition, OTUs specific for cases and controls were also quantified. We then studied the diversity at the overall number of reads level: 75% of all reads were similar between controls and cases, whereas the specific reads were restricted to the remaining 25%. At the phylum level, Proteobacteria represented 80-90% of all phyla in the blood, both in controls and cases. Within the Proteobacteria phylum we analyzed the differences at the family and genus level; Burkholderiaceae and Pseudomonadaceae represented 40% of the total families and Pseudomonas and Ralstonia represented 40% of the total genera. No major qualitative differences were observed between the taxons from controls and cases, even at the genus level. We then represented the diversity of the taxons identified in a dendrogram analysis. The semiquantitative representation of the phylogenic tree for each group of patients is shown in red and blue when cases and controls, respectively, were the most represented for each subdivision of the phylogenic tree, black when no difference occurred. When both dendrograms were matched, data showed that Proteobacteria diversity was the most preponderant and superimposable between both groups of patients. However, the main difference resides in the Firmicutes which seem to show a higher diversity in cases compared to controls. The opposite observation is made for the Bacteroidetes phylum.

At the quantitative level, 357 sequences of bacterial bacterial 16S rDNA are identical in blood from healthy patients and diabetic patients (for the quota of 10% difference). 121 and 128 sequences of bacterial bacterial 16S rDNA are respectively specific for patients who will remain non-diabetic throughout the monitoring and for patients who will develop diabetes after 6 years of monitoring.

Proteobacteria phylum represents the vast majority of the microbiota in the two groups of patients. Proteobacteria phylum is represented in greater proportion in patients who will develop diabetes after 6 years of monitoring (Table 1).

**Table 1 : % of each Phylum in the blood of healthy patients and diabetic patients.**

| **Phyla** | **Healthy %** | **Developed diabetes (6-9years) %** |
|---|---|---|
| Proteobacteria | 85,28 | 88,6 |
| Bacteroidetes | 6,96 | 5,82 |
| Actinobacteria | 2,33 | 2,17 |
| Firmicutes | 1,29 | 1,27 |
| Acidobacteria | 0,29 | 0,17 |
| TG-1 | 0,05 | 0,04 |
| TM6 | 0,01 | 0,01 |
| TM7 | 0,03 | 0,02 |
| Chloroflexi | 0 | 0,01 |
| Cyanobacteria | 0,23 | 0,17 |
| Fusobacteria | 0,01 | 0,01 |
| Planctomycetes | 0,01 | 0,02 |
| Unknown | 1,8 | 1,09 |

Within the Proteobacteria phylum, families *Burkhloderiaceae* and *Pseudomonaceae* are represented in greater proportion in patients who will develop diabetes after 6 years of monitoring in comparison to healthy patients (Table 2).

**Table 2: % of each family of the Proteobacteria Phylum in the blood of healthy patients and diabetic patients.**

| **Proteobacteria families** | **Healthy** | **Developed diabetes (6-9years)** |
|---|---|---|
| Caulobacteraceae | 3,81 | 2,02 |
| Bradyrhizobiaceae | 2,25 | 1,58 |
| Sphingomonadaceae | 1,52 | 0,81 |
| Burkholderiaceae | 35,09 | 39,38 |
| Comamonadaceae | 3,52 | 3,54 |
| Oxalobacteraceae | 6,45 | 6,31 |
| Moraxellaceae | 7,01 | 6,09 |
| Pseudomonadaceae | 34,23 | 35,78 |
| Xanthomonadaceae | 1,05 | 0,81 |

### Example 2: Identification of bacteria phyla and genera associated with obesity in the white adipose tissue of patients.

To identify phyla and genera associated with obesity, 16S rDNA quantification and sequencing were performed on adipose tissue from thin patients, i.e. patients who not developed obesity throughout the period of monitoring and from patients who developed obesity throughout the period of monitoring. Adipose tissue from each group of patient was obtained by trans-epidermal liposuction and 16S rDNA quantification and sequencing were performed in the same manner as in example 1.

An average 600, 400 or 300 different sequences are found in the adipose tissue of thin patients while about the double is detected in obese patients demonstrating that bacterial diversity is more abundant in adipose tissue from patients who developed obesity than in adipose tissue from thin patients.

150 sequences of bacterial 16S rDNA are identical in adipose tissue from thin patients and obese patients (considering that two sequences are different if less than 10% of their sequences differ from each other) and, 43 and 174 sequences of bacterial 16S rDNA are specific for the thin patients and obese patients respectively.

Firmicute phylum is represented in greater proportion in patients who developed obesity during the period of monitoring.

Within the Firmicute Phylum, genera *Streptococcaceae, Lactobacillaceae* and *Staphylococcaceae* are represented in greater proportion in patients who developed obesity during the period of monitoring.

### Example 3: Identification of bacteria phyla and genera associated with overweight, obesity and morbide obesity in the vascular stroma of adipose tissue of patients.

To identify phyla and genera associated with overweight, 16S rDNA quantification and sequencing were performed on the vascular stroma of white adipose tissue from thin patients, from overweight patients, from obese patients and from morbidly obese patients. Adipose tissue from each group of patient was obtained by trans-cutaneous liposuction and 16S rDNA quantification and sequencing were performed in the same manner as in example 1.

An average 500, 400, 200 different sequences are found in the vascular stroma from adipose tissue of thin patients while about 30% more is detected in overweight or obese patients demonstrating that bacterial diversity is more abundant in vascular stroma of adipose tissue from patients who developed obesity or overweight than in vascular stroma of adipose tissue from thin patients.

91 sequences of bacterial bacterial 16S rDNA are identical in thin patients and overweight patients (considering that two sequences are different if less than 10% of their sequences differ from each other) and, 26, 76, 51 and 56 sequences of bacterial 16S rDNA are respectively specific for the thin patients, overweight patients, obese patients and morbidly obese patients.

Firmicute and Proteobacteria phyla are represented in greater proportion in overweight, obese and morbidly obese patients (Table 3).

**Table 3: % of each Phylum in the stroma vascular fraction of adipose tissue in healthy patients, overweight, obese and morbidly obese patients.**

| **Phyla** | **Healthy** | **Overweight** | **Obese** | **Morbidly obese** |
|---|---|---|---|---|
| Acidobacteria | 0,00 | 0,02 | 0,00 | 0,05 |
| Actinobacteria | 0,90 | 1,62 | 1,23 | 0,88 |
| BD1-5 | 0,01 | 0,03 | 0,03 | 0,01 |
| Bacteroidetes | 1,38 | 1,83 | 1,77 | 1,26 |
| Candidate_division_OD1 | 0,00 | 0,03 | 0,02 | 0,01 |
| Candidate_division_SR1 | 0,00 | 0,00 | 0,00 | 0,00 |
| Candidate_division_TG-1 | 0,00 | 0,00 | 0,00 | 0,00 |
| Candidate_division_TM6 | 0,01 | 0,00 | 0,00 | 0,01 |
| Candidate_division_TM7 | 0,02 | 0,01 | 0,03 | 0,02 |
| Chlorobi | 0,00 | 0,00 | 0,00 | 0,01 |
| Chloroflexi | 0,01 | 0,00 | 0,00 | 0,01 |
| Cyanobacteria | 0,07 | 0,11 | 0,07 | 0,08 |
| Firmicutes | 84,02 | 78,06 | 53,71 | 72,33 |
| Fusobacteria | 0,01 | 0,00 | 0,00 | 0,00 |
| Nitrospirae | 0,00 | 0,00 | 0,00 | 0,00 |
| Planctomycetes | 0,00 | 0,00 | 0,00 | 0,00 |
| Proteobacteria | 13,08 | 17,82 | 42,68 | 25,00 |

Within the Firmicute phylum :
- *Lactobacillaceae, Leuconostocaceae* and *Veillonellaceae* families are represented in greater proportion in overweight patients, and *Clostridiaceae* family is represented in lesser proportion in overweight patients (Table 4),
- *Streptococcaceae* and *Staphylococcaceae* families are represented in greater proportion in obese patients, and *Clostridiaceae* and *Veillonellaceae* families are represented in lesser proportion in obese patients (Table 4),
- *Staphylococcaceae, Clostridiaceae* and *Leuconostocaceae* families are represented in greater proportion in morbidly obese patients, and *Streptococcaceae* and *Veillonellaceae* families are represented in lesser proportion in morbidly obese patients (Table 4).

**Table 4: % of each families of the Firmicutes Phylum in the stroma vascular fraction of adipose tissue in healthy patients, overweight, obese and morbidly obese patients.**

| **Firmicutes Families** | **Healthy** | **Overweight** | **Obese** | **Morbidly obese** |
|---|---|---|---|---|
| Alicyclobacillaceae | 0 | 0 | 0,0146339 | 0 |
| Bacillaceae | 0,0129153 | 0,0308244 | 0,0313584 | 0,00606916 |
| Family_XII_Incertae_Sedis | 0,00645765 | 0 | 0 | 0 |
| Family_XI_Incertae_Sedis | 0,019373 | 0 | 0,00418113 | 0 |
| Paenibacillaceae | 0,00968648 | 0,03783 | 0,0146339 | 0,0151729 |
| Planococcaceae | 0,00645765 | 0 | 0 | 0,00151729 |
| Staphylococcaceae | 0,132382 | 0,494592 | 1,01392 | 0,391461 |
| Aerococcaceae | 0 | 0,0140111 | 0,0271773 | 0 |
| Carnobacteriaceae | 0,00322883 | 0,0182144 | 0,00627169 | 0,0273112 |
| Enterococcaceae | 0,487553 | 0,550636 | 0,37003 | 0,34139 |
| Lactobacillaceae | 0,952504 | 3,9105 | 0,834135 | 0,819336 |
| Leuconostocaceae | 32,8888 | 52,0134 | 33,7062 | 36,3345 |
| Streptococcaceae | 11,2395 | 11,8478 | 13,6284 | 10,0551 |
| Clostridiaceae | 28,3071 | 1,03822 | 23,0213 | 32,3319 |
| Family_XIII_Incertae_Sedis | 0,00968648 | 0 | 0 | 0,00758645 |
| Family_XI_Incertae_Sedis | 0 | 0,0112089 | 0,102438 | 0,0121383 |
| Lachnospiraceae | 0,00645765 | 0,304041 | 0,0229962 | 0,010621 |
| Peptococcaceae | 0 | 0,00280222 | 0 | 0 |
| Peptostreptococcaceae | 0,00968648 | 0 | 0 | 0,010621 |
| Ruminococcaceae | 0,0129153 | 0,0420333 | 0,0292679 | 0 |
| Veillonellaceae | 3,24497 | 4,22014 | 2,38951 | 2,32297 |
| Erysipelotrichaceae | 0,019373 | 0,0154122 | 0,0167245 | 0,0121383 |

Within the Proteobacteria phylum *Burkhloderiaceae* and *Oxalobacteriaceae* families are represented in greater proportion in overweight, obese and morbidly obese patients, and *Neisseriaceae, Enterobacteriaceae* and *Moraxellaceae* families are represented in lesser proportion in overweight, obese and morbidly obese patients (Table 5).

**Table 5: % of each families of the Proteobacteria Phylum in the stroma vascular fraction of adipose tissue in healthy patients, overweight, obese and morbidly obese patients.**

| **Proteobacteria Families** | **Healthy** | **Overweight** | **Obese** | **Morbidly obese** |
|---|---|---|---|---|
| Caulobacteraceae | 0,0622019 | 0,460405 | 0,110494 | 0,171225 |
| Bradyrhizobiaceae | 0,020734 | 0,104359 | 0,0841862 | 0,021952 |
| Brucellaceae | 0 | 0,0429711 | 0,0342006 | 0,021952 |
| Hyphomicrobiaceae | 0 | 0,0184162 | 0,00526163 | 0,021952 |
| Methylobacteriaceae | 0,10367 | 0,374463 | 0,0789245 | 0,122931 |
| Methylocystaceae | 0,041468 | 0,0675261 | 0,0105233 | 0,0307328 |
| Nordella | 0 | 0 | 0,00263082 | 0 |
| Phyllobacteriaceae | 0,020734 | 0 | 0,00789245 | 0 |
| Rhizobiaceae | 0 | 0,0982198 | 0,126279 | 0,0131712 |
| Rhodobacteraceae | 0,186606 | 0,128913 | 0,115756 | 0,105369 |
| Acetobacteraceae | 0,020734 | 0 | 0,00789245 | 0,00439039 |
| Rhodospirillaceae | 0 | 0,00613874 | 0 | 0,0175616 |
| wr0007 | 0 | 0,0122775 | 0,00263082 | 0 |
| Candidatus Captivus | 0 | 0 | 0 | 0,00878079 |
| Candidatus Midichloria | 0 | 0 | 0,00263082 | 0,00439039 |
| Candidatus Odvssella | 0 | 0,00613874 | 0 | 0 |
| Erythrobacteraceae | 0,020734 | 0,0306937 | 0,0368314 | 0,0395135 |
| GOBB3-C201 | 0 | 0 | 0,0184157 | 0,0131712 |
| Sphingomonadaceae | 0,20734 | 0,380602 | 0,35253 | 0,373183 |
| Alcaligenaceae | 0,228074 | 0,184162 | 0,115756 | 0,149273 |
| Burkholderiaceae | 15,7993 | 29,9877 | 62,895 | 48,4875 |
| Comamonadaceae | 5,39084 | 5,69675 | 3,33851 | 5,36067 |
| Oxalobacteraceae | 5,88845 | 13,1185 | 7,74513 | 7,52074 |
| Methylophilaceae | 0,041468 | 0,0552486 | 0,0710321 | 0,0351232 |
| Neisseriaceae | 4,37487 | 2,08717 | 1,1102 | 2,19959 |
| Nitrosomonadaceae | 0 | 0,00613874 | 0 | 0 |
| Rhodocyclaceae | 0,290276 | 0,276243 | 0,16048 | 0,149273 |
| Bacteriovoraceae | 0 | 0,0122775 | 0 | 0 |
| Bdellovibrionaceae | 0 | 0,0429711 | 0,00263082 | 0,00439039 |
| Desulfovibrionaceae | 0 | 0,00613874 | 0,0105233 | 0 |
| 0319-6G20 | 0,31101 | 0,411295 | 0,0736629 | 0,184397 |
| Cystobacterineae | 0,020734 | 0,0122775 | 0 | 0 |
| Sorangiineae | 0,124404 | 0,0491099 | 0,0157849 | 0,021952 |
| mle1-27 | 0 | 0,0245549 | 0 | 0,0131712 |
| Campylobacteraceae | 0,622019 | 0,245549 | 0,113125 | 0,105369 |
| Aeromonadaceae | 1,67945 | 1,2523 | 0,586672 | 0,636607 |
| Alteromonadaceae | 0 | 0,116636 | 0 | 0 |
| Chromatiaceae | 0 | 0,0429711 | 0 | 0 |
| Enterobacteriaceae | 33,6098 | 20,8533 | 10,4338 | 16,4157 |
| Coxiellaceae | 0 | 0,00613874 | 0 | 0,00878079 |
| Legionellaceae | 0,041468 | 0,239411 | 0,0157849 | 0,0482943 |
| Oceanospirillaceae | 0 | 0,00613874 | 0 | 0,021952 |
| Pasteurellaceae | 0,020734 | 0,0306937 | 0,00526163 | 0,021952 |
| Moraxellaceae | 24,5283 | 15,1504 | 7,83457 | 11,5599 |
| Pseudomonadaceae | 3,21377 | 4,94168 | 3,23327 | 3,99526 |
| Sinobacteraceae | 0 | 0,135052 | 0,0920786 | 0,0746367 |
| Xanthomonadaceae | 0,31101 | 0,441989 | 0,25782 | 0,342451 |

Within the Proteobacteria phylum *Ralstonia* and *Undibacterium* genera are represented in greater proportion in overweight, obese and morbidly obese patients, and *Neisseriales, Enteric_Bacteria_Cluster, Enhydrobacter* and *Acinetobacter* genera are represented in lesser proportion in overweight, obese and morbidly obese patients (Table 6),

**Table 6: % of each genera of the Proteobacteria Phylum in the stroma vascular fraction of adipose tissue in healthy patients, overweight, obese and morbidly obese patients.**

| **Proteobacteria genera** | **Healthy** | **Overweight** | **Obese** | **Morbidly obese** |
|---|---|---|---|---|
| Asticcacaulis | 0 | 0,00613874 | 0,00263082 | 0 |
| Brevundimonas | 0,041468 | 0,380602 | 0,0684013 | 0,127321 |
| Caulobacter | 0,020734 | 0,0736648 | 0,00789245 | 0,0263424 |
| Bradyrhizobium | 0,020734 | 0,092081 | 0,0552472 | 0,021952 |
| Rhodopseudomonas | 0 | 0,00613874 | 0,0210465 | 0 |
| Ochrobactrum | 0 | 0,0184162 | 0 | 0 |
| Devosia | 0 | 0,00613874 | 0 | 0,00878079 |
| Hyphomicrobium | 0 | 0,0122775 | 0 | 0,0131712 |
| Pedomicrobium | 0 | 0 | 0,00263082 | 0 |
| Methylobacterium | 0,10367 | 0,374463 | 0,0789245 | 0,122931 |
| Pleomorphomonas | 0 | 0,00613874 | 0,00263082 | 0,0175616 |
| Defluvibacter | 0,020734 | 0 | 0 | 0 |
| Mesorhizobium | 0 | 0 | 0,00263082 | 0 |
| Phyllobacterium | 0 | 0 | 0,00263082 | 0 |
| Rhizobium | 0 | 0,0982198 | 0,126279 | 0,0131712 |
| Amaricoccus | 0 | 0 | 0,00263082 | 0 |
| Paracoccus | 0,041468 | 0,0368324 | 0,0105233 | 0,0175616 |
| Rhodobacter | 0 | 0,0306937 | 0 | 0 |
| Rubellimicrobium | 0,020734 | 0 | 0 | 0 |
| Roseomonas | 0 | 0 | 0,00789245 | 0 |
| Azospirillum | 0 | 0 | 0 | 0,0131712 |
| Telmatospirillum | 0 | 0 | 0 | 0,00439039 |
| Porphyrobacter | 0,020734 | 0,0306937 | 0,0368314 | 0,0395135 |
| Novosphingobium | 0,0622019 | 0,0245549 | 0,00789245 | 0 |
| Sphingomonas | 0,10367 | 0,331492 | 0,344637 | 0,346841 |
| Sphingopyxis | 0,020734 | 0 | 0 | 0 |
| Achromobacter | 0,10367 | 0,0368324 | 0,0210465 | 0,0439039 |
| Alcaligenes | 0,10367 | 0,0859423 | 0,0841862 | 0,0439039 |
| Castellaniella | 0 | 0 | 0 | 0,0131712 |
| GKS98 | 0 | 0 | 0 | 0,00878079 |
| Parasutterella | 0 | 0,0491099 | 0 | 0 |
| Burkholderia | 0,456148 | 0,202578 | 0,347268 | 0,144883 |
| Cupriavidus | 0,10367 | 0,0675261 | 0,00789245 | 0,00878079 |
| Polynucleobacter | 0,041468 | 0,0245549 | 0,0631396 | 0,00878079 |
| Ralstonia | 15,198 | 29,5457 | 62,4714 | 48,3075 |
| Acidovorax | 2,61248 | 1,53468 | 0,752414 | 1,54542 |
| Aquabacterium | 0 | 0,92081 | 0,0184157 | 0,00439039 |
| Aquamonas | 0,020734 | 0 | 0,00526163 | 0 |
| Comamonas | 0,539084 | 0,33763 | 0,165741 | 0,381964 |
| Curvibacter | 0 | 0 | 0 | 0,00878079 |
| Delftia | 0,020734 | 0,0122775 | 0,0157849 | 0 |
| Diaphorobacter | 0,31101 | 0,399018 | 0,0920786 | 0,149273 |
| Hydrogenophaga | 0 | 0,0245549 | 0,0105233 | 0,00439039 |
| Methylibium | 0 | 0 | 0 | 0,00878079 |
| Pelomonas | 0,912295 | 1,60221 | 1,97048 | 2,92839 |
| Polaromonas | 0 | 0,0184162 | 0 | 0,00878079 |
| Rhodoferax | 0,145138 | 0,184162 | 0,102602 | 0,0526847 |
| Schlegelella | 0 | 0 | 0,00789245 | 0 |
| Variovorax | 0 | 0,0122775 | 0,00526163 | 0 |
| Duganella | 0,20734 | 0,0982198 | 0,121018 | 0,0658559 |
| Herbaspirillum | 0,041468 | 0,00613874 | 0 | 0,0175616 |
| Herminiimonas | 0 | 0 | 0,00789245 | 0 |
| Janthinobacterium | 0,248808 | 0,122775 | 0,0394623 | 0,0790271 |
| Massilia | 0,0622019 | 0,135052 | 0 | 0,00439039 |
| Oxalobacter | 0 | 0 | 0 | 0,00439039 |
| Undibacterium | 5,20423 | 12,3634 | 7,53729 | 7,25732 |
| Methylobacillus | 0 | 0,0245549 | 0 | 0,00439039 |
| Methylophilus | 0,041468 | 0,0122775 | 0,0342006 | 0,021952 |
| Aquitalea | 0,10367 | 0,092081 | 0,0368314 | 0,0307328 |
| Deefgea | 0 | 0 | 0 | 0,00439039 |
| Neisseria | 0 | 0 | 0,00263082 | 0,00878079 |
| Neisseriales | 3,87725 | 1,70043 | 0,80503 | 1,87031 |
| Azoarcus | 0 | 0 | 0,00789245 | 0 |
| Azospira | 0 | 0 | 0 | 0,0131712 |
| Dechloromonas | 0 | 0,092081 | 0 | 0 |
| Methyloversatilis | 0,020734 | 0,0122775 | 0 | 0,00878079 |
| Thauera | 0,041468 | 0 | 0,0210465 | 0,0395135 |
| Uliginosibacterium | 0,10367 | 0,0368324 | 0,0210465 | 0,0263424 |
| Zoogloea | 0,124404 | 0,135052 | 0,110494 | 0,0482943 |
| Bacteriovorax | 0 | 0,0122775 | 0 | 0 |
| Bdellovibrio | 0 | 0,0429711 | 0 | 0,00439039 |
| OM27 | 0 | 0 | 0,00263082 | 0 |
| Desulfovibrio | 0 | 0,00613874 | 0,0105233 | 0 |
| Myxococcaceae | 0,020734 | 0 | 0 | 0 |
| Polyangiaceae | 0,10367 | 0,0429711 | 0,0157849 | 0,021952 |
| Arcobacter | 0,165872 | 0,184162 | 0,0368314 | 0,0658559 |
| Sulfurospirillum | 0,41468 | 0,0552486 | 0,0736629 | 0,0395135 |
| Aeromonas | 1,53431 | 1,06814 | 0,457762 | 0,526847 |
| Tolumonas | 0,0829359 | 0,171885 | 0,12891 | 0,10976 |
| OM60(NOR5) clade | 0 | 0,116636 | 0 | 0 |
| Alishewanella | 0 | 0,0368324 | 0 | 0 |
| Rheinheimera | 0 | 0,00613874 | 0 | 0 |
| Enteric Bacteria cluster | 33,5891 | 20,8042 | 10,3865 | 16,3586 |
| Morganella | 0,020734 | 0,0491099 | 0,0447239 | 0,0395135 |
| Proteus | 0 | 0 | 0,00263082 | 0 |
| Rickettsiella | 0 | 0,00613874 | 0 | 0,00878079 |
| Legionella | 0,041468 | 0,227133 | 0,0157849 | 0,0263424 |
| Marinomonas | 0 | 0 | 0 | 0,021952 |
| Pseudospirillum | 0 | 0,00613874 | 0 | 0 |
| Haemophilus | 0,020734 | 0,0306937 | 0,00526163 | 0,021952 |
| Acinetobacter | 22,289 | 13,6955 | 6,83486 | 10,2823 |
| Enhydrobacter | 2,23927 | 1,43646 | 0,99708 | 1,24687 |
| Psychrobacter | 0 | 0 | 0 | 0,0263424 |
| Azomonas | 0 | 0 | 0 | 0,0131712 |
| Cellvibrio | 0,020734 | 0,116636 | 0,00263082 | 0,00878079 |
| Pseudomonas | 3,19303 | 4,82505 | 3,22801 | 3,97331 |
| Nevskia | 0 | 0,128913 | 0,0552472 | 0 |
| Arenimonas | 0 | 0,0429711 | 0,0105233 | 0 |
| Rhodanobacter | 0,020734 | 0 | 0,00526163 | 0,00439039 |
| Stenotrophomonas | 0,269542 | 0,380602 | 0,231512 | 0,33367 |
| Thermomonas | 0 | 0,00613874 | 0 | 0 |

### Example 4: Use of bacterial flora for the demonstration of a concept of protection against the development of metabolic diseases through vaccination.

In order to induce an immunization process which could involve a resistance to the effect of fat diet on the development of metabolic diseases, the intestinal bacterial flora has been inactivated and then administered by intraperitoneal route to mice in a repetitive manner.

We identified that during a fat diet, bacteria of the intestine are translocated in the adipose tissue and then induce an inflammatory response which is responsible for the development of the adipose tissue leading to obesity and for the resistance to the insulin leading to diabetes.

We demonstrated that repeated injections of intestinal bacteria which translocated in the adipose tissue allowed healthy mice to resist to development of impaired glucose tolerance (IGT) and then to diabetes (Figures 1 and 6) and weight gain (Figure 5). This protection persists over time up to 131 days, although it is diminishing (Figures 2 to 4). A booster vaccination would probably be desirable to prolong the effect.

It has been demonstrated that a fat-enriched diet induces diabetes and modifies the bacterial phylotypes from the intestinal microbiota (metagenome). Therefore it is suggested that this change corresponded to a change in the corresponding antigenic repertoire. Hence a single subcutaneous administration to mice of bacterial extracts from the ileum of diabetic mice has been shown to be able to reduce the impact of a diabetogenic diet (high fat diet) on the vaccinated mouse. The protection was shown using intraperitoneal glucose tolerance test. It was analyzed 19, 30,38 and 66 days following the beginning of the diabetogenic diet. The effects of the subcutaneous administration of bacterial extracts prepared in the same condition but issued from normal chow fed non diabetic mice were compared. The bacterial extract from normal mice could not protect against high fat diet induced diabetes (Figures 7 to 14).

The treatment of donor mice with antibiotics also prevented the efficacy of the bacterial extracts to reduce the impact of a fat-enriched diet on a vaccinated mouse. This suggests that the ileum extract should contain bacteria to efficiently vaccinate the mice. Eventually it has been demonstrated that the vaccinated process did indeed involve a lymphocyte mediated immune response since the transfer of the lymphocytes from the spleen of the vaccinated mouse does protect the recipient mouse from high fat diet induced diabetes (Figures 15 to 17). Importantly this was also observed for the control of the fat to lean mass ratio (Figure 18).

## Claims

1. A method for identifying a phylum, family and/or genus of bacteria, which is a prognostic marker of diabetes, which method comprises the steps consisting of :
a) quantifying bacteria in a biological sample from a subject healthy at the time of sampling and who developed diabetes, within a ten year period after sampling;
b) comparing said quantified bacteria of step a) with the bacteria from a control; and
c) identifying phyla, families and/or genera of bacteria which are present in different proportions within the sample assayed in step a) and the control;
wherein said identified phyla, families and/or genera of bacteria which are present in different proportions are prognostic markers of diabetes, wherein the control refers to a subject healthy at the time of sampling who did not develop diabetes within a ten year period of sampling and,
wherein said biological sample of step a) and the control sample is blood.

2. A method for identifying a phylum, family and/or genus of bacteria, which is a prognostic marker of overweight, obesity and/or morbide obesity, which method comprises the steps consisting of :
a) quantifying bacteria in a biological sample from a subject healthy at the time of sampling and who developed overweight, obesity and/or morbide obesity, within a ten year period after sampling;
b) comparing said quantified bacteria of step a) with the bacteria from a control; and
c) identifying phyla, families and/or genera of bacteria which are present in different proportions within the sample assayed in step a) and the control;
wherein said identified phyla, families and/or genera of bacteria which are present in different proportions are prognostic markers of overweight, obesity and/or morbide obesity, wherein the control refers to a subject healthy at the time of sampling who did not develop overweight, obesity and/or morbide obesity within a ten year period of sampling and,
wherein said biological sample of step a) and the control sample is adipose subcutaneous tissue, or vascular stroma of adipose subcutaneous tissue.

3. An *in vitro* method for prognosing diabetes, which method comprises the steps consisting of :
a) quantifying bacteria in a biological sample from a subject healthy at the time of sampling;
b) comparing said quantified bacteria of step a) with the bacteria from a control; and
c) identifying phyla, families and/or genera of bacteria which are prognostic markers of diabetes and which are present in different proportions within the sample of the individual assayed in step a) and the control;
wherein said presence in different proportions indicates that the subject is at risk of developing diabetes,
wherein the control refers to a subject healthy at the time of sampling who did not develop diabetes within a ten year period of sampling and,
wherein said biological sample of step a) and the control sample are blood.

4. The method according to claim 3, wherein the identification of bacteria of the Proteobacteria phylum in step c) indicates that the subject is at risk of developing diabetes.

5. An *in vitro* method for prognosing overweight, obesity and/or morbide obesity, which method comprises the steps consisting of :
a) quantifying bacteria in a biological sample from a subject healthy at the time of sampling;
b) comparing said quantified bacteria of step a) with the bacteria from a control; and
c) identifying phyla, families and/or genera of bacteria which are prognostic markers of overweight, obesity and/or morbide obesity and which are present in different proportions within the sample of the individual assayed in step a) and the control;
wherein said presence in different proportions indicates that the subject is at risk of developing overweight, obesity and/or morbide obesity,
wherein the control refers to a subject healthy at the time of sampling who did not develop overweight, obesity and/or morbide obesity within a ten year period of sampling and,
wherein said biological sample of step a) and the control sample is adipose subcutaneous tissue, or vascular stroma of adipose subcutaneous tissue.

6. The method according to claim 5, wherein the identification of bacteria of the Proteobacteria phylum in step c) indicates that the subject is at risk of developing overweight, obesity and/or morbide obesity.

7. The method according to claim 3, wherein the identification of bacteria of the *Burkhloderiaceae* and/or *Pseudomonaceae* families of the Proteobacteria phylum in step c) indicates that the subject is at risk of developing diabetes.

8. The method according to claim 5, wherein the identification of bacteria of the Firmicute phylum in step c) indicates that the subject is at risk of developing, overweight, obesity and/or morbide obesity.

9. The method according to claim 5, wherein the identification of bacteria of the *Burkhloderiaceae, Oxalobacteriaceae, Neisseriaceae, Enterobacteriaceae* and/or *Moraxellaceae* families of the Proteobacteria phylum and/or *Streptococcaceae, Lactobacillaceae, Staphylococcaceae, Clostridiaceae* and/or *Veillonellaceae* of the Firmicutes phylum in step c) indicates that the subject is at risk of developing obesity.

10. The method according to claim 5, wherein the identification of bacteria of the Firmicute phylum in step c) indicates that the subject is at risk of developing overweight.

11. The method according to claim 5, wherein the identification of bacteria of the *Burkhloderiaceae, Oxalobacteriaceae, Neisseriaceae, Enterobacteriaceae* and/or *Moraxellaceae* families of the Proteobacteria phylum and/or *Lactobacillaceae, Leuconostocaceae, Clostridiaceae* and/or *Veillonellaceae* families of the Firmicutes phylum in step c) indicates that the subject is at risk of developing overweight.

12. The method according to claim 5, wherein the identification of bacteria of the *Ralstonia, Undibacterium, Neisseriales, Enteric Bacteria Cluster, Enhydrobacter* and/or *Acinetobacter* genera of the Proteobacteria phylum in step c) indicates that the subject is at risk of developing overweight, obesity and/or morbide obesity.

13. The method according to anyone of claims 1 to 12, wherein said quantification of bacteria is performed by quantifying the amount of bacterial 16S rDNA.

14. The method according to claim 12, wherein said quantification of bacteria is performed by quantifying the amount of bacterial 16S rDNA by quantitative polymerase chain reaction.

## Patentansprüche

1. Verfahren zur Identifizierung eines Stamms, einer Familie und/oder Gattung von Bakterien, der/die ein prognostischer Marker für Diabetes ist, wobei das Verfahren die folgenden Schritte umfasst:
a) Quantifizierung von Bakterien in einer biologischen Probe aus einem Subjekt, das zum Zeitpunkt der Probenentnahme gesund war und innerhalb eines Zeitraums von zehn Jahren nach der Probenentnahme Diabetes entwickelte;
b) Vergleichen der quantifizierten Bakterien von Schritt a) mit den Bakterien aus einer Kontrolle; und
c) Identifizierung von Stämmen, Familien und/oder Gattungen von Bakterien, die innerhalb der in Schritt a) untersuchten Probe und der Kontrolle in unterschiedlichen Proportionen vorhanden sind;
wobei besagte identifizierte Stämmen, Familien und/oder Gattungen von Bakterien, die in unterschiedlichen Proportionen vorhanden sind, prognostische Marker von Diabetes sind, wobei sich die Kontrolle auf ein Subjekt bezieht, das zum Zeitpunkt der Probenahme gesund war und innerhalb eines Zeitraums von zehn Jahren nach der Probenentnahme kein Diabetes entwickelte, und
wobei es sich bei der biologischen Probe von Schritt a) und bei der Kontrollprobe um Blut handelt.

2. Verfahren zur Identifizierung eines Stamms, einer Familie und/oder einer Gattung von Bakterien, der/die ein prognostischer Marker für Übergewicht, Fettleibigkeit und/oder morbide Fettleibigkeit ist, wobei das Verfahren die folgenden Schritte umfasst:
a) Quantifizierung von Bakterien in einer biologischen Probe aus einem Subjekt, das zum Zeitpunkt der Probenentnahme gesund war und innerhalb eines Zeitraums von zehn Jahren nach der Probenentnahme Übergewicht, Fettleibigkeit und/oder morbide Fettleibigkeit entwickelte;
b) Vergleichen der quantifizierten Bakterien von Schritt a) mit den Bakterien aus einer Kontrolle; und
c) Identifizierung von Stämmen, Familien und/oder Gattungen von Bakterien, die innerhalb der in Schritt a) untersuchten Probe und der Kontrolle in unterschiedlichen Proportionen vorhanden sind;
wobei besagte identifizierte Stämmen, Familien und/oder Gattungen von Bakterien, die in unterschiedlichen Proportionen vorhanden sind, prognostische Marker von Übergewicht, Fettleibigkeit und/oder morbide Fettleibigkeit sind, wobei sich die Kontrolle auf ein Subjekt bezieht, das zum Zeitpunkt der Probenahme gesund war und innerhalb eines Zeitraums von zehn Jahren nach der Probenentnahme kein Übergewicht, keine Fettleibigkeit und/oder keine morbide Fettleibigkeit entwickelte, und
wobei es sich bei der biologischen Probe von Schritt a) und bei der Kontrollprobe um adipöses, subkutanes Gewebe oder vaskuläres Stützgewebe von adipösem, subkutanem Gewebe handelt.

3. *In-vitro*-Verfahren zur Prognose von Diabetes, wobei das Verfahren die folgenden Schritte umfasst:
a) Quantifizierung von Bakterien in einer biologischen Probe aus einem Subjekt, das zum Zeitpunkt der Probenentnahme gesund war;
b) Vergleichen der quantifizierten Bakterien von Schritt a) mit den Bakterien aus einer Kontrolle; und
c) Identifizierung von Stämmen, Familien und/oder Gattungen von Bakterien, die prognostische Marker von Diabetes sind und die innerhalb der Probe von dem in Schritt a) untersuchten Subjekt und der Kontrolle in unterschiedlichen Proportionen vorhanden sind;
wobei besagte Gegenwart unterschiedlicher Proportionen andeutet, dass das Subjekt ein Risiko aufweist, Diabetes zu entwickeln, wobei sich die Kontrolle auf ein Subjekt bezieht, das zum Zeitpunkt der Probenahme gesund war und innerhalb eines Zeitraums von zehn Jahren nach der Probenentnahme kein Diabetes entwickelte, und
wobei es sich bei der biologischen Probe von Schritt a) und bei der Kontrollprobe um Blut handelt.

4. Verfahren nach Anspruch 3, wobei die Identifizierung von Bakterien des Proteobakterium-Stamms in Schritt c) andeutet, dass das Subjekt gefährdet ist, Diabetes zu entwickeln.

5. *In-vitro*-Verfahren zur Prognose von Übergewicht, Fettleibigkeit und/oder morbider Fettleibigkeit, wobei das Verfahren die folgenden Schritte umfasst:
a) Quantifizierung von Bakterien in einer biologischen Probe aus einem Subjekt, das zum Zeitpunkt der Probenentnahme gesund war;
b) Vergleichen der quantifizierten Bakterien von Schritt a) mit den Bakterien aus einer Kontrolle; und
c) Identifizierung von Stämmen, Familien und/oder Gattungen von Bakterien, die prognostische Marker von Übergewicht, Fettleibigkeit und/oder morbider Fettleibigkeit sind und die innerhalb der Probe von dem in Schritt a) untersuchten Subjekt und der Kontrolle in unterschiedlichen Proportionen vorhanden sind;
wobei besagte Gegenwart unterschiedlicher Proportionen andeutet, dass das Subjekt ein Risiko aufweist, Übergewicht, Fettleibigkeit und/oder morbide Fettleibigkeit zu entwickeln, wobei sich die Kontrolle auf ein Subjekt bezieht, das zum Zeitpunkt der Probenahme gesund war und innerhalb eines Zeitraums von zehn Jahren nach der Probenentnahme kein Übergewicht, keine Fettleibigkeit und/oder keine morbide Fettleibigkeit entwickelte, und
wobei es sich bei der biologischen Probe von Schritt a) und bei der Kontrollprobe um adipöses, subkutanes Gewebe oder vaskuläres Stützgewebe von adipösem, subkutanem Gewebe handelt.

6. Verfahren nach Anspruch 5, wobei die Identifizierung von Bakterien des Proteobakterium-Stamms in Schritt c) andeutet, dass das Subjekt gefährdet ist, Übergewicht, Fettleibigkeit und/oder morbide Fettleibigkeit zu entwickeln.

7. Verfahren nach Anspruch 3, wobei die Identifizierung von Bakterien der *Burkhloderiaceae-* und/oder *Pseudomonaceae*-Familien des Proteobakterien-Stammes in Schritt c) andeutet, dass das Subjekt gefährdet ist, Diabetes zu entwickeln.

8. Verfahren nach Anspruch 5, wobei die Identifizierung von Bakterien des Firmicute-Stamms in Schritt c) andeutet, dass das Subjekt gefährdet ist, Übergewicht, Fettleibigkeit und/oder morbide Adipositas zu entwickeln.

9. Verfahren nach Anspruch 5, wobei die Identifizierung von Bakterien der *Burkhloderiaceae-, Oxalobacteriaceae-, Neisseriaceae-, Enterobacteriaceae-* und/oder *Moraxellaceae*-Familien des Proteobakterien-Stamms und/oder *Streptokokken, Lactobacillaceae, Staphylococcaceae, Clostridiaceae* und/oder *Veillonellaceae* des Firmicutes-Stamms in Schritt c) andeutet, dass das Subjekt gefährdet ist, Fettleibigkeit zu entwickeln.

10. Verfahren nach Anspruch 5, wobei die Identifizierung von Bakterien des Firmicutes-Stamms in Schritt c) andeutet, dass das Subjekt gefährdet ist, Übergewicht zu entwickeln.

11. Verfahren nach Anspruch 5, wobei die Identifizierung von Bakterien der *Burkhloderiaceae-, Oxalobacteriaceae-, Neisseriaceae-, Enterobacteriaceae-* und/oder *Moraxellaceae*-Familien des Proteobakterien-Stamms und/oder *Lactobacillaceae-, Leuconostocaceae-, Clostridiaceae-* und/oder *Veillonellaceae*-Familien des Firmicutes-Stamms in Schritt c) andeutet, dass das Subjekt gefährdet ist, Übergewicht zu entwickeln.

12. Verfahren nach Anspruch 5, wobei die Identifizierung von Bakterien der *Ralstonia-, Undibacterium-, Neisseriales-,* Enteric_Bacteria_Cluster-*, Enhydrobacter-* und/oderAcinetobacter-Gattungen des Proteobakterien-Stamms in Schritt c) andeutet, dass das Subjekt gefährdet ist, Übergewicht, Fettleibigkeit und/oder morbide Fettleibigkeit zu entwickeln.

13. Verfahren nach einem der Ansprüche 1 bis 12, wobei die besagte Quantifizierung von Bakterien durch Quantifizierung der Menge an bakterieller 16S rDNA durchgeführt wird.

14. Verfahren nach Anspruch 12, wobei die besagte Quantifizierung von Bakterien durch Quantifizierung der Menge an bakterieller 16S rDNA durch quantitative Polymerase-Kettenreaktion durchgeführt wird.

## Revendications

1. Procédé pour identifier un embranchement, une famille et/ou un genre de bactéries, qui est un marqueur pronostique du diabète, lequel procédé comprend les étapes consistant à :
a) quantifier des bactéries dans un échantillon biologique d'un sujet en bonne santé au moment du prélèvement et qui a développé un diabète, dans une période de dix ans suivant le prélèvement ;
b) comparer lesdites bactéries quantifiées de l'étape a) avec les bactéries d'un contrôle ; et
c) identifier des embranchements, des familles et/ou des genres de bactéries qui sont présentes en différentes proportions dans l'échantillon analysé à l'étape a) et le contrôle ;
dans lequel lesdits embranchements, familles et/ou genres identifiés de bactéries qui sont présentes en différentes proportions sont des marqueurs pronostiques du diabète, où le contrôle fait référence à un sujet en bonne santé au moment du prélèvement qui n'a pas développé de diabète dans une période de dix ans suivant le prélèvement et,
dans lequel ledit échantillon biologique de l'étape a) et l'échantillon de contrôle sont du sang.

2. Procédé pour identifier un embranchement, une famille et/ou un genre de bactéries, qui est un marqueur pronostique du surpoids, de l'obésité et/ou de l'obésité morbide, lequel procédé comprend les étapes consistant à :
a) quantifier des bactéries dans un échantillon biologique d'un sujet en bonne santé au moment du prélèvement et qui a développé un surpoids, une obésité et/ou une obésité morbide, dans une période de dix ans suivant le prélèvement ;
b) comparer lesdites bactéries quantifiées de l'étape a) avec les bactéries d'un contrôle ; et
c) identifier des embranchements, des familles et/ou des genres de bactéries qui sont présentes en différentes proportions dans l'échantillon analysé à l'étape a) et le contrôle ;
dans lequel lesdits embranchements, familles et/ou genres identifiés de bactéries qui sont présentes en différentes proportions sont des marqueurs pronostiques du surpoids, de l'obésité et/ou de l'obésité morbide, où le contrôle fait référence à un sujet en bonne santé au moment du prélèvement qui n'a pas développé de surpoids, d'obésité et/ou d'obésité morbide dans une période de dix ans suivant le prélèvement et,
dans lequel ledit échantillon biologique de l'étape a) et l'échantillon de contrôle sont un tissu adipeux sous-cutané, ou un stroma vasculaire de tissu adipeux sous-cutané.

3. Procédé *in vitro* de pronostic du diabète, lequel procédé comprend les étapes consistant à :
a) quantifier des bactéries dans un échantillon biologique d'un sujet en bonne santé au moment du prélèvement ;
b) comparer lesdites bactéries quantifiées de l'étape a) avec les bactéries d'un contrôle ; et
c) identifier des embranchements, des familles et/ou des genres de bactéries qui sont des marqueurs pronostiques du diabète et qui sont présentes en différentes proportions dans l'échantillon de l'individu analysé à l'étape a) et le contrôle ;
dans lequel ladite présence en différentes proportions indique que le sujet est à risque de développer le diabète,
dans lequel le contrôle fait référence à un sujet en bonne santé au moment du prélèvement qui n'a pas développé de diabète dans une période de dix ans suivant le prélèvement et,
dans lequel ledit échantillon biologique de l'étape a) et l'échantillon de contrôle sont du sang.

4. Procédé selon la revendication 3, dans lequel l'identification de bactéries de l'embranchement Proteobacteria à l'étape c) indique que le sujet est à risque de développer le diabète.

5. Procédé *in vitro* de pronostic du surpoids, de l'obésité et/ou de l'obésité morbide, lequel procédé comprend les étapes consistant à :
a) quantifier des bactéries dans un échantillon biologique d'un sujet en bonne santé au moment du prélèvement ;
b) comparer lesdites bactéries quantifiées de l'étape a) avec les bactéries d'un contrôle ; et
c) identifier des embranchements, des familles et/ou des genres de bactéries qui sont des marqueurs pronostiques du surpoids, de l'obésité et/ou de l'obésité morbide et qui sont présentes en différentes proportions dans l'échantillon de l'individu analysé à l'étape a) et le contrôle ;
dans lequel ladite présence en différentes proportions indique que le sujet est à risque de développer un surpoids, une obésité et/ou une obésité morbide,
dans lequel le contrôle fait référence à un sujet en bonne santé au moment du prélèvement qui n'a pas développé de surpoids, d'obésité et/ou d'obésité morbide dans une période de dix ans suivant le prélèvement et,
dans lequel ledit échantillon biologique de l'étape a) et l'échantillon de contrôle sont un tissu adipeux sous-cutané, ou un stroma vasculaire de tissu adipeux sous-cutané.

6. Procédé selon la revendication 5, dans lequel l'identification de bactéries de l'embranchement Proteobacteria à l'étape c) indique que le sujet est à risque de développer un surpoids, une obésité et/ou une obésité morbide.

7. Procédé selon la revendication 3, dans lequel l'identification de bactéries des familles *Burkholderiaceae* et/ou *Pseudomonaceae* de l'embranchement Proteobacteria à l'étape c) indique que le sujet est à risque de développer le diabète.

8. Procédé selon la revendication 5, dans lequel l'identification de bactéries de l'embranchement Firmicutes à l'étape c) indique que le sujet est à risque de développer un surpoids, une obésité et/ou une obésité morbide.

9. Procédé selon la revendication 5, dans lequel l'identification de bactéries des familles *Burkholderiaceae, Oxalobacteraceae, Neisseriaceae, Enterobacteriaceae* et/ou *Moraxellaceae* de l'embranchement Proteobacteria et/ou *Streptococcaceae, Lactobacillaceae, Staphylococcaceae, Clostridiaceae* et/ou *Veillonellaceae* de l'embranchement Firmicutes à l'étape c) indique que le sujet est à risque de développer une obésité.

10. Procédé selon la revendication 5, dans lequel l'identification de bactéries de l'embranchement Firmicutes à l'étape c) indique que le sujet est à risque de développer un surpoids.

11. Procédé selon la revendication 5, dans lequel l'identification de bactéries des familles *Burkholderiaceae, Oxalobacteraceae, Neisseriaceae, Enterobacteriaceae* et/ou *Moraxellaceae* de l'embranchement Proteobacteria et/ou *Lactobacillaceae, Leuconostocaceae, Clostridiaceae* et/ou *Veillonellaceae* de l'embranchement Firmicutes à l'étape c) indique que le sujet est à risque de développer un surpoids.

12. Procédé selon la revendication 5, dans lequel l'identification de bactéries des genres *Ralstonia, Undibacterium, Neisseriales, Enteric Bacteria Cluster, Enhydrobacter* et/ou *Acinetobacter* de l'embranchement Proteobacteria à l'étape c) indique que le sujet est à risque de développer un surpoids, une obésité et/ou une obésité morbide.

13. Procédé selon l'une quelconque des revendications 1 à 12, dans lequel ladite quantification des bactéries est réalisée en quantifiant la quantité d'ADNr 16S bactérien.

14. Procédé selon la revendication 12, dans lequel ladite quantification des bactéries est réalisée en quantifiant la quantité d'ADNr 16S bactérien par une réaction en chaîne par polymérase quantitative.


### SEQUENCE LISTING

<110> INSTITUT NATIONAL DE LA SANTE ET DE LA RECHERCHE MEDICALE (INSERM) CHU DE TOULOUSE
<120> USE OF BROOD OR TISSUE BACTERIOME FOR PREDICTION, DIAGNOSIS AND PREVENTION OF METABOLIC DISEASES AND THEIR CARDIOVASCULAR COMPLICATIONS
<130> BET12P0531
<150> EP 11305366.4
   <151> 2011-03-31
<160> 6
<170> PatentIn version 3.5
<210> 1
   <211> 19
   <212> DNA
   <213> Homo sapiens
<400> 1
   tcctacggga ggcagcagt 19
<210> 2
   <211> 26
   <212> DNA
   <213> Homo sapiens
<400> 2
   ggactaccag ggtatctaat cctgtt 26
<210> 3
   <211> 18
   <212> DNA
   <213> Homo sapiens
<400> 3
   cggtgaatac gttcccgg 18
<210> 4
   <211> 22
   <212> DNA
   <213> Homo sapiens
<400> 4
   tacggctacc ttgttacgac tt 22
<210> 5
   <211> 20
   <212> DNA
   <213> Homo sapiens
<400> 5
   agagtttgat cmtggctcag 20
<210> 6
   <211> 18
   <212> DNA
   <213> Homo sapiens
<400> 6
   gtcgcctccc gtaggagt 18